(19) Europäisches Patentamt
European Patent Office
Office européen des brevets

(11) Veröffentlichungsnummer: **0 023 332**
**A2**

(12) # EUROPÄISCHE PATENTANMELDUNG

(21) Anmeldenummer: **80104266.4**

(22) Anmeldetag: **19.07.80**

(51) Int. Cl.$^3$: **C 07 C 149/23**
C 07 C 153/09, C 07 C 154/00
C 07 C 154/02, C 07 C 155/04
C 07 D 307/22, C 07 D 307/52
C 07 D 333/36, A 01 N 37/00
A 01 N 43/00, A 01 N 47/00

(30) Priorität: **25.07.79 DE 2930145**
**03.04.80 DE 3013104**

(43) Veröffentlichungstag der Anmeldung:
**04.02.81 Patentblatt 81/5**

(84) Benannte Vertragsstaaten:
**AT BE CH DE FR GB IT LI LU NL SE**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Koch, Manfred, Dr.**
**Kreuzheck 2**
**D-6239 Eppstein/Taunus(DE)**

(72) Erfinder: **Mildenberger, Hilmar, Dr.**
**Fasanenstrasse 24**
**D-6233 Kelkheim (Taunus)(DE)**

(72) Erfinder: **Sachse, Burkhard, Dr.**
**An der Ziegelei 30**
**D-6233 Kelkheim (Taunus)(DE)**

(54) **Fungizide Thioglykolsäureanilide, Verfahren zu ihrer Herstellung und diese enthaltende Schädlingsbekämpfungsmittel.**

(57) Gegenstand der Erfindung sind neue Thioglykolsäureanilide der Formel I,

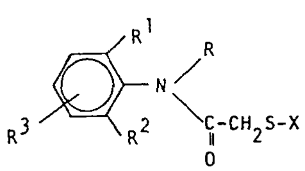

(1)

worin $R^4$

R = -CHCOR$^5$, 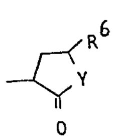

X = 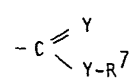 , 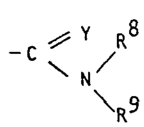 , 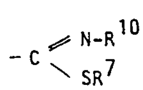

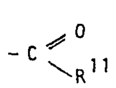 , Wasserstoff

$R^1$ = $(C_1$-$C_4)$-Alkyl, Halogen, $(C_1$-$C_3)$-Alkylthio, $(C_1$-$C_4)$-Alkoxy, $(C_3$-$C_4)$-Alkenyl

$R^2$ = Wasserstoff, $(C_1$-$C_4)$-Alkyl, Halogen

$R^3$, $R^4$, $R^6$ = Wasserstoff, Methyl

$R^5$ = Hydroxy, $(C_1$-$C_4)$-Alkoxy, $(C_1$-$C_3)$-Alkylthio, $(C_1$-$C_2)$-Alkoxy -$(C_1$-$C_2)$-alkoxy, Amino, $(C_1$-$C_2)$-Alkylamino, Di-$(C_1$-$C_2)$-alkylamino

Y = *Sauerstoff oder Schwefel*

$R^7$ = gegebenenfalls substituiertes $(C_1$-$C_6)$-Alkyl, $(C_3$-$C_6)$-Cycloalkyl, $(C_3$-$C_4)$-Alkenyl, $(C_3$-$C_4)$-Alkinyl

$R^8$ = Wasserstoff, $(C_1$-$C_4)$-Alkyl

$R^9$ = gegebenenfalls substituiertes $(C_1$-$C_4)$-Alkyl, $(C_3$-$C_4)$-Alkenyl, Furfuryl, Tetrahydrofurfuryl, mit der Maßgabe, daß für Y=S *nicht* beide Reste $R^8$ und $R^9$ $(C_1$-$C_4)$-Alkyl bedeuten

$R^{10}$ = -CN, gegebenenfalls substituiertes $(C_1$-$C_4)$-Alkyl, Allyl, Furfuryl,

$R^{11}$ = Wasserstoff, gegebenenfalls substituiertes $(C_1$-$C_4)$-Alkyl $(C_2$-$C_4)$-Alkenyl, mit der Maßgabe, daß $R^{11}$ im Falle von

R = 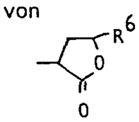 nicht für $(C_1$-$C_4)$-Alkyl oder halogen-substituiertes $(C_1$-$C_4)$-Alkyl steht, bedeuten, ferner Verfahren zu deren Herstellung sowie deren Verwendung als Schädlingsbekämpfungsmittel.

EP 0 023 332 A2

HOECHST AKTIENGESELLSCHAFT HOE 79/F 206 K Dr.GM/St

# Fungizide Thioglykolsäureanilide, Verfahren zu ihrer Herstellung und diese enthaltende Schädlingsbekämpfungsmittel

Gegenstand der Erfindung sind neue Thioglykolsäureanilide, Verfahren zu ihrer Herstellung sowie ihre Verwendung als Schädlingsbekämpfungsmittel, insbesondere im Pflanzenschutz.

Die erfindungsgemäßen Verbindungen besitzen die allgemeine Formel I,

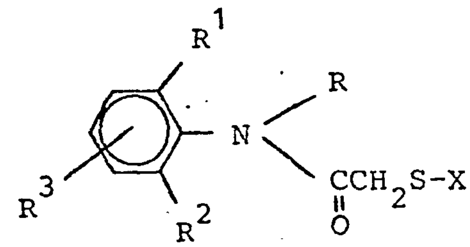

(I)

worin

R = 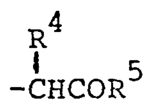 , 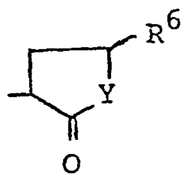

X = 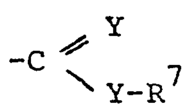 , 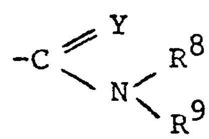 , 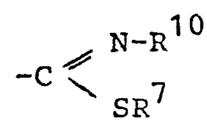 ,

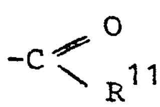 , Wasserstoff

$R^1$ = $(C_1-C_4)$-Alkyl, Halogen, $(C_1-C_3)$-Alkylthio, $(C_1-C_4)$-Alkoxy, $(C_3-C_4)$-Alkenyl

$R^2$ = Wasserstoff, $(C_1-C_4)$-Alkyl, Halogen

$R^3$, $R^4$, $R^6$ = Wasserstoff, Methyl

$R^5$ = Hydroxy, $(C_1-C_4)$-Alkoxy, $(C_1-C_3)$-Alkylthio, $(C_1-C_2)$-Alkoxy-$(C_1-C_2)$alkoxy, Amino, $(C_1-C_2)$-Alkylamino, Di- $(C_1-C_2)$-alkylamino

Y = Sauerstoff oder Schwefel

$R^7$ = $(C_1-C_6)$-Alkyl, welches durch $(C_1-C_2)$-Alkoxy oder Halogen substituiert sein kann, $(C_3-C_6)$-Cycloalkyl, $(C_3-C_4)$-Alkenyl, $(C_3-C_4)$-Alkinyl

$R^8$ = Wasserstoff, $(C_1-C_4)$-Alkyl

$R^9$ = $(C_1-C_4)$-Alkyl, welches gegebenenfalls durch Halogen, $(C_1-C_2)$-Alkoxy substituiert ist, $(C_3-C_4)$-Alkenyl, Furfuryl, Tetrahydrofuryl mit der Maßgabe, daß für Y = S nicht beide Reste $R^8$ und $R^9$ $(C_1-C_4)$-Alkyl bedeuten

$R^{10}$= -CN, $(C_1-C_4)$-Alkyl, welches gegebenenfalls durch Halogen oder $(C_1-C_2)$-Alkoxy substituiert ist, Allyl, Furfuryl

$R^{11}$= Wasserstoff, gegebenenfalls durch ein- bis drei Halogenatome, $(C_1-C_2)$-Alkoxy oder $(C_1-C_2)$-Alkylthio substituiertes $(C_1-C_4)$-Alkyl, $(C_2-C_4)$-Alkenyl, mit der Maßgabe, daß $R^{11}$ im Falle von R= 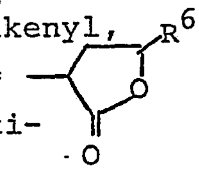 nicht für $(C_1-C_4)$-Alkyl oder halogensubstituiertes $(C_1-C_4)$-Alkyl steht, bedeutet.

Bevorzugte Reste in Formel I sind:

$R^1$= Methyl, Äthyl, Isopropyl, Chlor oder Brom

$R^2$= Wasserstoff, Methyl,

$R^3$, $R^4$, $R^6$= Wasserstoff, Methyl

$R^5$= Methoxy, Äthoxy, Propoxy, Isopropoxy, Methylthio, Äthylthio

Y = Sauerstoff oder Schwefel

$R^7$= $(C_1-C_6)$-Alkyl, welches durch Methoxy, Äthoxy oder Chlor substituiert sein kann, Allyl, Butenyl, Propargyl, $(C_3-C_6)$-Cycloalkyl

$R^8$= Wasserstoff, $(C_1-C_4)$-Alkyl

$R^9$= $(C_1-C_4)$-Alkyl, welches gegebenenfalls durch $(C_1-C_2)$-Alkoxy substituiert ist, $(C_3-C_4)$-Alkenyl, Furfuryl, mit der Maßgabe, daß für Y=S nicht beide Reste $R^8$ und $R^9$ $(C_1-C_4)$-Alkyl bedeuten

$R^{10}$= -CN, $(C_1-C_4)$-Alkyl, welches gegebenenfalls durch $(C_1-C_2)$-Alkoxy substituiert ist, Allyl, Furfuryl

$R^{11}$= Wasserstoff, gegebenenfalls durch ein- bis drei Halogenatome, $(C_1-C_2)$-Alkoxy oder $(C_1-C_2)$-Alkylthio substituiertes $(C_1-C_4)$-Alkyl, $(C_2-C_4)$-Alkenyl, mit

der Maßgabe, daß $R^{11}$ im Falle von R= 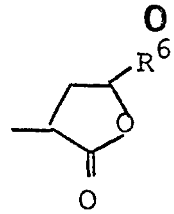 nicht für $(C_1-C_4)$-Alkyl oder halogensubstituiertes $(C_1-C_4)$-Alkyl steht.

Es ist bekannt, zur Bekämpfung von Fäulniserkrankungen an Pflanzen, welche durch Pilze der Gattung Phytophthora verursacht werden, Fungizide aus der Reihe der Dithiocarbamate, wie zum Beispiel Mancozeb oder Maneb, oder Perchlormethylmerkaptanderivate, wie zum Beispiel Captafol, zu verwenden. Mit diesen Wirkstoffen können die oberirdisch an Blatt- und Stengelwerk angreifenden Phytophthorastämme bekämpft werden, während bodenbürtige Erreger der Gattung Phytophthora und Pythium, welche an den Wurzeln der Pflanzen angreifen, nicht erfaßt werden. Es ist weiter bekannt, daß acylierte Anilinoessig-, bzw. -propionsäurederivate, wie sie in den DE-Offenlegungsschriften 23 50 944, 26 43 445, 25 13 730, 25 13 789, 25 15 091 und 26 43 477 beschrieben sind, neben einer herbiziden Wirksamkeit auch eine fungizide Wirkung gegen Pilze der Familie der Oomyceten besitzen.

Ester der Thio- oder Dithiokohlensäure sind dagegen als landwirtschaftlich verwertbare Wirkstoffe bisher nur in wenigen Fällen (z.B. als Herbizide) bekannt geworden (DE-AS 10 31 571, FR-PS 11 71 404).

Es wurde nun überraschenderweise gefunden, daß die erfindungsgemäßen Thioglykolsäureanilide der Formel I eine hervorragende fungizide Wirkung, insbesondere gegen die der Klasse der Phycomyceten angehörenden Oomyceten, wie zum Beispiel Pythium, Phytophthora, Peronospora, Pseudoperonospora und Plasmopara aufweisen. Daneben wirken sie auch gegen Pilze der Klassen Ascomycetes, Basidiomycetes (z.B. Rostpilze) und Fungi imperfecti. Damit können Pilze an den verschiedensten Kulturpflanzen, wie zum Beispiel Mais, Reis, Getreide, Zuckerrüben, Gemüse, Gurkengewächse, Kartoffeln, Tomaten, Reben, Hopfen, Tabak, Zitrus- und

Paprikaarten, Zierpflanzen, Kakao, Bananen und Kautschuk bekämpft bzw. gehemmt bzw. ihr Auftreten an diesen Pflanzen ganz verhindert werden. Die Verbindungen der Formel I wirken teilweise systemisch. Sie lassen sich auch als Beizmittel zur Bekämpfung von samenbürtigen Pilzen an Saatgut oder zur Bekämpfung der im Erdboden auftretenden phytopathogenen Pilze einsetzen.

Die Wirkung der erfindungsgemäßen Verbindungen der Formel I ist u.a. auch deshalb als überaus überraschend zu bezeichnen, weil entsprechende Alkylthioacetanilide, wie sie in der oben genannten DE-OS 25 15 091 beschrieben sind, gegen Phytophthora, insbesondere gegen bodenbürtige Phytophthoraarten und Pilze der Familie Pythium nur eine sehr geringe fungizide Wirkung aufweisen.

Gegenstand der Erfindung sind daher auch fungizide Mittel, die gekennzeichnet sind durch einen Gehalt an einer Verbindung der Formel I.

Die Herstellung von Verbindungen der Formel I ist nach verschiedenen Methoden möglich und kann zum Beispiel nach einem der nachstehend aufgeführten Verfahren erfolgen:

a) Durch Umsetzung einer Verbindung der allgemeinen Formel II,

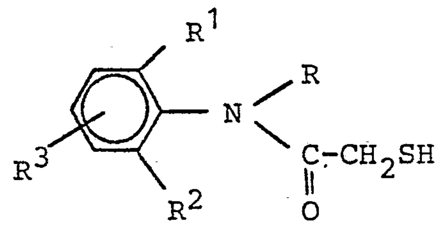

(II)

in der R, $R^1$, $R^2$ und $R^3$ die Bedeutungen wie in Formel I haben, in Abhängigkeit vom Rest X in Formel I mit

$a_1$) Säurehalogeniden der allgemeinen Formel III,

Hal-X (III)

in der X die Bedeutung wie in Formel I hat und vorzugsweise

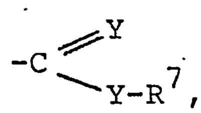

,

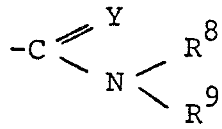 (für $R^8 \neq H$), 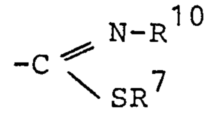 ,

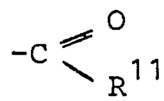 bedeutet, wobei die Reste Y, $R^7$, $R^8$, $R^9$, $R^{10}$ und $R^{11}$ die Bedeutungen wie in Formel I haben und Hal für Halogen, vorzugsweise Chlor oder Brom steht, und wobei anstelle des Säurehalogenids Hal-X der Formel III gegebenenfalls auch das entsprechende Säureanhydrid der Formel IIIa,

X - O - X (IIIa)

worin X die Bedeutung wie in Formel III hat, eingesetzt werden kann,

oder

$a_2$) für Verbindungen der Formel I mit X = 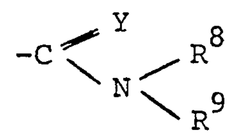

und $R^8$ = H, durch Umsetzung mit Isocyanaten der Formel IIIb,

$YCN — R^9$ (IIIb)

worin Y und $R^9$ die Bedeutungen wie in Formel I haben, gemäß folgendem Reaktionsschema:

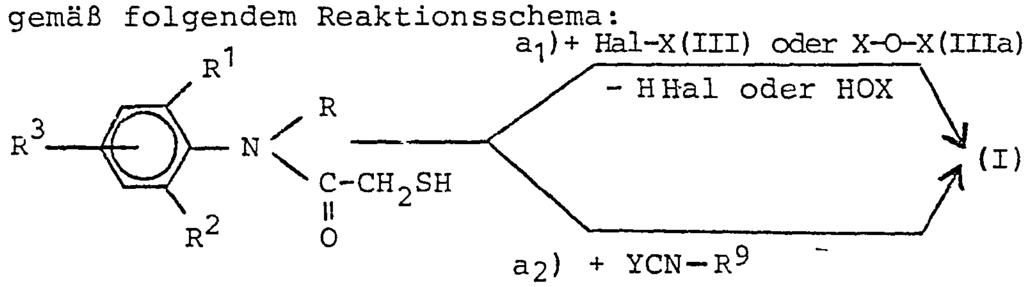


(II)

b) Durch Umsetzung einer Verbindung der allgemeinen Formel IV,

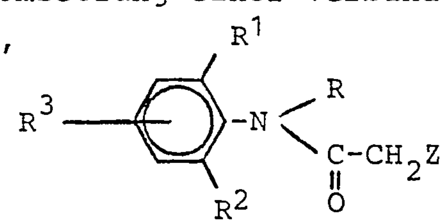 (IV)

in der R, $R^1$, $R^2$, $R^3$ die Bedeutungen wie in Formel I haben und Z einen nukleophil substituierbaren Abgangsrest, vorzugsweise aus der Gruppe Halogen, Tosylat oder Mesylat, insbesondere Chlor oder Brom, bedeutet, mit einer Verbindung der allgemeinen Formel V,

$$MeS - X \qquad (V)$$

in der X die Bedeutung wie in Formel I hat und Me ein anorganisches oder organisches Kation, vorzugsweise Natrium, Kalium, 1/2-Calcium, 1/2-Magnesium oder Ammonium bedeutet, gemäß folgendem Reaktionsschema:

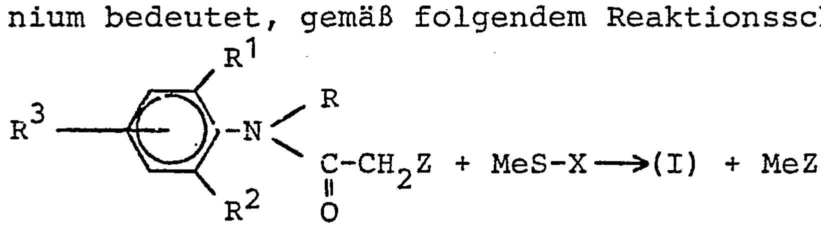

(IV) (V)

c) Durch Acylierung von Anilinderivaten der allgemeinen Formel VI,

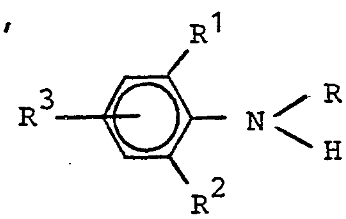

(VI)

in der R, $R^1$, $R^2$ und $R^3$ die Bedeutungen wie in Formel I haben, mit Säurehalogeniden oder Säureanhydriden der allgemeinen Formel VII,

$$W - \overset{O}{\overset{\|}{C}} - CH_2 - SX \qquad (VII)$$

in der X die Bedeutung wie in Formel I hat und W ein Halogenatom, vorzugsweise Chlor oder Brom, oder den Rest $XS-CH_2-\overset{O}{\overset{\|}{C}}-O-$, worin X die Bedeutung wie in Formel VII hat, bedeutet, gemäß folgendem Reaktionsschema:

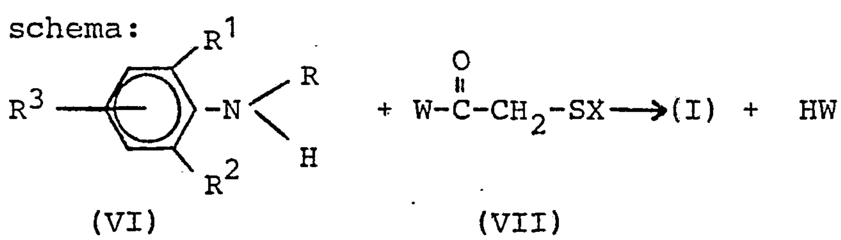

(VI) (VII)

Nach dieser Verfahrensweise lassen sich bevorzugt Verbindungen der Formel I mit

X = 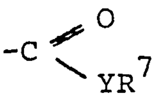 , 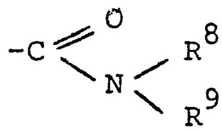 mit $R^8 \neq H$,

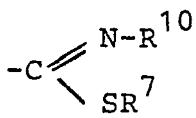 , 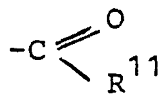

herstellen.

d) Für Verbindungen der Formel I mit X = 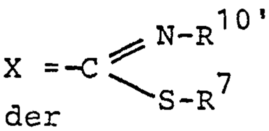 durch Alkylierung von Verbindungen der allgemeinen Formel VIII,

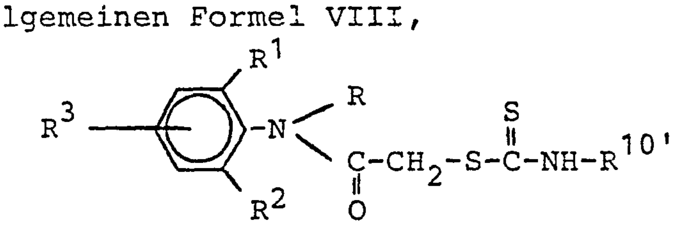

(VIII)

in der R, $R^1$, $R^2$ und $R^3$ die Bedeutungen wie in Formel I haben und $R^{10'}$ für $(C_1-C_4)$-Alkyl, welches gegebenenfalls durch $(C_1-C_2)$-Alkoxy substituiert ist, Allyl oder Furfuryl steht, mit Halogeniden oder Sulfaten der Formel IX,

$$Q - R^7 \qquad (IX)$$

in der $R^7$ die Bedeutung wie in Formel I hat und Q Halogen oder $R^7O-SO_2-O-$ , vorzugsweise Chlor, Brom oder Jod, bedeutet, gemäß folgendem Reaktionsschema:

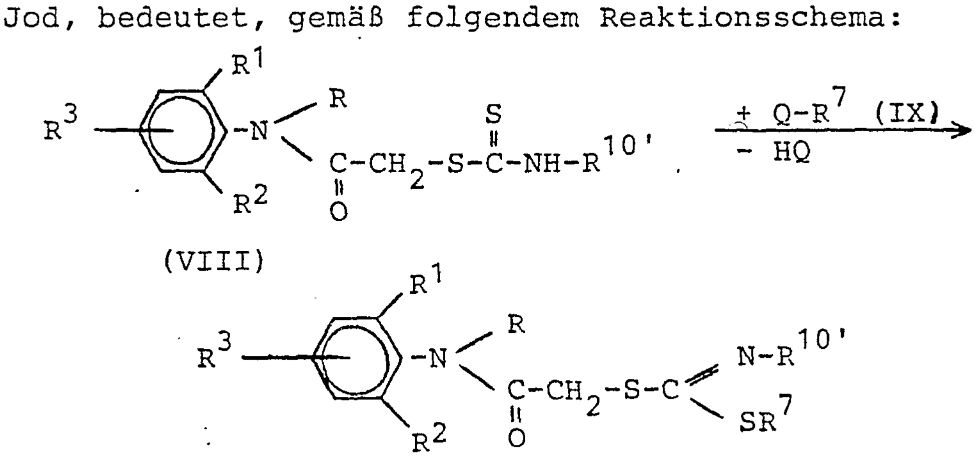


(VIII)

(I)

Die Umsetzungen können in An- oder Abwesenheit von gegenüber den Reaktanten inerten Lösungs- oder Verdünnungsmitteln durchgeführt werden. Beispielsweise kommen als Lösungsmittel in Frage: aliphatische, aromatische oder araliphatische Kohlenwasserstoffe wie Benzol, Toluol, Xylol, Petroläther, halogenierte Kohlenwasserstoffe wie Chlorbenzol, Methylenchlorid, Äther wie Dialkyläther, Dioxan oder Tetrahydrofuran, Nitrile wie Acetonitril, dialkylierte Amide wie Dimethylformamid, Ketone wie Aceton oder Methyläthylketon und im Fall einer Herstellung nach einer der Methoden b) oder d) außerdem auch Alkohole wie Methanol, Äthanol, Äthylenglykol oder Wasser sowie Gemische solcher Lösungsmittel.

Die Reaktionstemperaturen sind nicht kritisch und liegen zwischen -20 und +180°C, vorzugsweise zwischen 0 und 120°C.

Die Umsetzungen nach den Methoden $a_1$), c) und d) können in An- oder Abwesenheit von säurebindenden Mitteln durchgeführt werden, die Umsetzung nach Methode $a_2$) kann durch Zusatz von Basen, wie zum Beispiel Pyridin oder Triäthylamin katalysiert werden. Als säurebindende Mittel kommen zum Beispiel in Frage: Trialkylamine wie Triäthylamin, Pyridin, anorganische Basen wie z.B. Metalloxide oder Metallhydroxyde, beispielsweise Calziumoxyd oder Natriumhydroxyd oder Carbonate wie Kaliumcarbonat.

Werden die Umsetzungen nach den Methoden $a_1$) oder c) ohne Zusatz von säurebindenden Mitteln durchgeführt, wird vorteilhaft bei erhöhter Temperatur gearbeitet und z.B. der sich bildende Halogenwasserstoff aus dem Reaktionsgemisch abgeführt.

Wird die Herstellung der Verbindungen der Formel I in Lösungsmitteln durchgeführt, in denen einer der Reaktanten nur wenig löslich ist, z.B. bei Verwendung von unpolaren Lösungsmitteln wie Kohlenwasserstoffen oder Wasser, so

kann eine Beschleunigung der Umsetzung durch Zugabe von Katalysatoren, wie z.B. Kronenäthern oder quarternären Ammonium- bzw. Phosphoniumsalzen erreicht werden.

Die Herstellung von Verbindungen der Formel II erfolgt nach an sich bekannten Methoden, wie sie z.B. in Houben-Weyl, Methoden der organischen Chemie, Bd. 9, 4. Auflage (1955) S. 12 - 18 beschrieben sind. Eine bevorzugte Herstellungsweise steht in der reduktiven Spaltung von Disulfiden der Formel X, z.B. mittels Zink, zur Verfügung, gemäß folgendem Reaktionsschema:

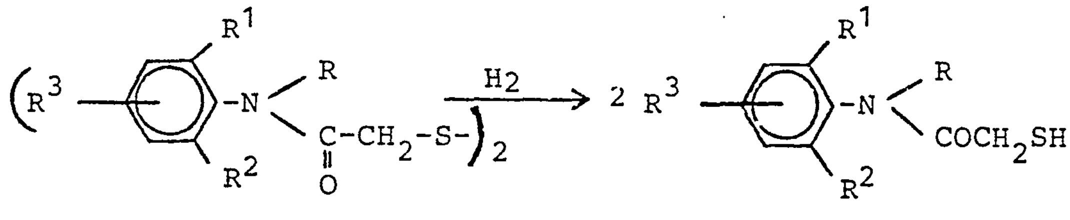


(X) (II)

Die Herstellung der als Ausgangsmaterial verwendeten Anilinderivate der Formel VI kann zum Beispiel erfolgen nach: J.Org.Chem. 30, 4101 (1965); DE-OS 22 12 268; US-PS 40 12 519; Tetrahedron 1967, 487 und 493.

Aus den Anilinderivaten der Formel VI können durch Acylierung zum Beispiel mittels Chloracetylchlorid oder Bromacetylchlorid die Ausgangsprodukte der Formel IV hergestellt werden.

Säurehalogenide der Formel III, Isocyanate der Formel IIIb und Verbindungen der Formel V sind bekannt. (vgl. Houben-Weyl, Methoden der organischen Chemie, Bd. 9, 4. Auflage (1955), S. 808 - 809, S. 813-16, S. 820, S. 829, S. 835, S. 838, S. 842-46, S. 849)

Säurehalogenide der Formel VII können nach dem Fachmann geläufigen Methoden, z.B. ausgehend von Thioglykolsäure oder Chloressigsäure, hergestellt werden.

Die Verbindungen der Formel I mit R = 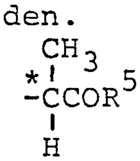 und 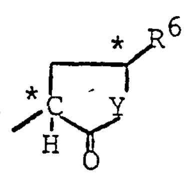

besitzen asymmetrische Kohlenstoffatome. Sie liegen daher im allgemeinen als racemische Gemische enantiomerer Formen vor. Die enantiomeren Formen weisen eine unterschiedliche biologische Aktivität auf. Im Falle von $R = -\overset{CH_3}{\underset{*}{C}}HCOR^5$ weisen die Verbindungen der Formel I der D-(oder R)-Konfiguration eine gegenüber dem Racemat und der L- (oder S)-Konfiguration erhöhte Wirksamkeit auf. Eine Reihe von Verbindungen der Formel I mit D-Konfiguration sind als Beispiele genannt. Sie werden von den racemischen Verbindungen durch den Zusatz "AD" unterschieden. Eine gezielte Synthese des reinen D-Isomeren kann zum Beispiel durch fraktionierte Kristallisation von Salzen der Anilinopropionsäure der allgemeinen Formel XI mit optisch aktiven Basen

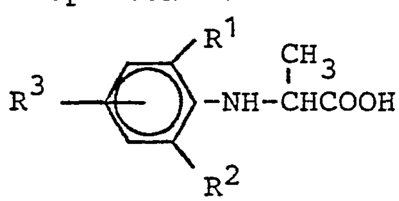

(XI)

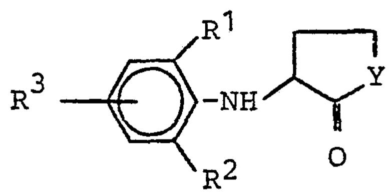

(XIa)

wie Cinchonin oder $\alpha$-Phenylethylamin oder durch fraktionierte Kristallisation von Anilinobutyrolactonen der allgemeinen Formel XIa mit optisch aktiven Säuren, wie zum Beispiel D- oder L-Weinsäure erfolgen. In den Formeln XI und XIa haben $R^1$, $R^2$, $R^3$ und Y die Bedeutungen wie in Formel I.

Es ist aber auch eine gezielte Synthese, ausgehend von optisch aktiven Halogenpropionsäurederivaten, wie zum Beispiel L-2-Chlorpropionsäuremethylester, optisch aktiven Milchsäureestersulfonaten oder optisch aktiven Halogenbutyrolactonen und Anilinen der allgemeinen Formel XII,

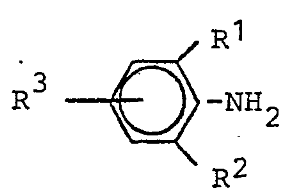

(XII)

in der $R^1$, $R^2$ und $R^3$ die Bedeutung wie in Formel I haben, möglich. Diese Umsetzung erfolgt vorwiegend als SN2-Reaktion unter Inversion der Konfiguration. So entstehen aus 2,6-Dimethylanilin und L-2-Chlorpropionsäure bzw. L-2-Chlorpropionsäuremethylester bevorzugt D-2-(2,6-Dimethylanilino)-propionsäure bzw. deren Methylester.

Die erfindungsgemäßen Mittel enthalten die Wirkstoffe der Formel I im allgemeinen zu etwa 2-95 Gew.-%, vorzugsweise 5-90 Gew.-%. Sie können als Spritzpulver, emulgierbare Konzentrate, versprühbare Lösungen, Stäubemittel , Beizmittel Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, zum Beispiel polyoxethylierte Alkylphenole, polyoxethylierte Fettalkohole, Alkyl- oder Alkylphenyl-sulfonate und Dispergiermittel, zum Beispiel ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise, zum Beispiel durch Mahlen und Vermischen der Komponenten.

Emulgierbare Konzentrate können zum Beispiel durch Auflösen des Wirkstoffes in einem inerten organischen Lösungsmittel, zum Beispiel Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:

Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol—Propylenoxid-Ethylenoxid-Kondensationsprodukte, Alkylpolyglykolether, Sorbitanfettsäureester, Polyoxethylensorbitan-fettsäureester oder Polyoxethylen-sorbitester.

Stäubemittel kann man durch Vermahlen des Wirkstoffes mit fein verteilten, festen Stoffen, zum Beispiel Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln , zum Beispiel Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - granuliert werden.

In Spritzpulvern beträgt die Wirkstoffkonzentration zum Beispiel etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%.

Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel-, Füll- oder Trägerstoffe.

Zur Anwendung werden die in handelsüblicher Form vorliegenden Konzentrate gegebenenfalls in üblicher Weise verdünnt, zum Beispiel bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen pestiziden Wirkstoffen, wie zum Beispiel Insektiziden, Akaraziden, Herbiziden, Wachstumsregulatoren oder Fungiziden sind gegebenenfalls möglich. Insbesondere bei Mischungen mit Fungiziden können teilweise auch synergistische Wirkungssteigerungen erzielt werden. Als mögliche fungizide Mischungspartner können beispielsweise folgende genannt werden:

Elementarer Schwerfel
Ammoniumpolysulfid
Natriumpolysulfid
Bariumpolysulfid
Calciumpolysulfid und Calciumthiosulfat
Calciumhypochlorit
Borsäure
Natriumtetraborat-dekahydrat (BORAX)
Zinkchlorid
Magnesiumborat
Nickelsulfat
Kaliumchromat
Bleiarsenat
Cadmiumchlorid
Cadmiumcarbonat
Kupfer(I)oxyd (KUPFEROXID)
Bordeaux-Brühe
Kupfer(II)sulfat-pentahydrat (KUPFERSULFAT)
Basisches Kupfer(II)chlorid (KUPFEROXICHLORID)
Kupfer(II)phosphat
Tribasisches Kupfer(II)sulfat (DREIBASISCHES KUPFERSULFAT)
Basisches Kupfer(II)carbonat (KUPFERCARBONAT)
Kupfer(II)-dihydrazin-sulfat
Kupferaminkomplexe
Kupfer(II)sulfat-Ammoniumcarbonat-Mischung
Kupfer(II)chlorid-basisches Kupfer(II)sulfat-Mischung
Basisches Kupfer(II)carbonat-Zinksalz-Mischung
Kupfer(II)-Zink-chromat-Komplex (KUPFER ZINK CHROMAT)
Kupfer(II)-Zink-cadium-calcium-chromat-Komplex
Kupfer(II)Salz der Ölsäure (KUPFEROLEAT)
Kupfer(II)salze von Fettsäuren
Kupfer(II)salz der Naphthensäure
Kupfer(II)salz des 8-Hydroxychinolins
Kupfer(II)salz des 1,2-Naphthochinonoxims-(2)
Kupfer(II)salz des 3-Phenylsalicylats
Bis-(tri-n-butylzinn)oxid
Triphenylzinnhydroxyd (MENTINHYDROXID)
Triphenylzinnacetat (FENTINACETAT)

Bis-(tributylzinn)succinat
Quecksilber(I)chlorid (KALOMEL)
Quecksilber(II)chlorid
Quecksilber(II)oxyd
Quecksilber-Zink-chromat-Komplex
Quecksilber(II)lactat
Äthylquecksilberchlorid
2-Hydroxyäthylquecksilberacetat
Äthylquecksilberisothiocyanat
3-Äthoxypropylquecksilberbromid
Chlormethoxypropylquecksilberacetat
Methoxyäthylquecksilberchlorid
2-Methoxyäthylquecksilbersilikat
Bis-(methylquecksilber)sulfat
Bis-(methylquecksilber)ammoniumacetat
Äthylquecksilberacetat
2-Methoxyäthylquecksilberacetat
Äthylquecksilberphosphat
Isopropylmethylquecksilberacetat
Methylquecksilbercyanid
Methylquecksilberbenzoat
N-Cyano-N'(methylquecksilber)guanidin
Methylquecksilberpentachlorphenolat
Äthylquecksilber-2,3-dihydroxypropylmerkaptid
Methylquecksilber-8-hydroxychinolat (Ortho LM)
N-(Methylquecksilber)-1,4,5,6,7,7-hexachlorobicyclo[2,2,1]hept-5-en-2,3-dicarboximid
N-(Äthylquecksilber)-1,4,5,6,7,7-hexachlorobicyclo[2,2,1]hept-5-en-2,3-dicarboximid
Natriumsalz des Äthylquecksilberthiosalicylats
N-(Äthylquecksilber)-p-toluolsulfonsäureanilid
Phenylquecksilberacetat (PAM)
Phenylquecksilberpropionat
Phenylquecksilbertriäthanolammoniumlactat (PAS)
Phenylquecksilberharnstoff
N-(Phenylquecksilber)-1,4,5,6,7,7-hexachlorobicyclo-[2,2,1]hept-5-en-2,3-dicarboximid
Phenylquecksilberdimethyldithiocarbamat

Phenylquecksilberformamid
Phenylquecksilberchlorid
Phenylquecksilberacetat
Phenylquecksilberbenzoat
Phenylquecksilberborat
Phenylquecksilberhydroxyd
Phenylquecksilberjodid
Basisches Phenylquecksilbernitrat
Phenylquecksilbermonoäthanolaminlactat
Phenylquecksilbersalicylat
Hydroxyquecksilberchlorphenol
Hydroxyquecksilbertrichlorphenol
Hydroxyquecksilbernitrophenol
N-Phenylquecksilberäthylendiamin
Phenylquecksilbermonoäthanolammoniumacetat
Pyridylquecksilberacetat
Diphenylquecksilber-8-hydroxychinolat
Quecksilber(II)-Komplex mit organischen Phosphaten
Mischung von Methylquecksilber-2,3-dihydroxypropylmerkaptid und Methylquecksilberacetat
Mischung von Äthylquecksilber-2,3-dihydroxypropylmerkaptid und Äthylquecksilberacetat
Mischung von Hydroxyquecksilberchlorphenol und Hydroxyquecksilbernitrophenol
Quecksilber-Cadmium-organischer Komplex

Cadmiumsuccinat
Cadmium-di-n-propyl-xanthogenat
Cadmium-8-hydroxychinolat
Phenylaminocadmiumacetat
Phenylaminocadmiumdilactat
Methylarsinsulfid
Zinkoktat
Zinkoleat
Formalin
Paraformaldehyd
Acrolein
Methylbromid

Methylisothiocyanat
Tetrajodäthylen
1,3-Dichlorpropen und verwandte chlorierte $C_3$-Kohlenwasserstoffe
1-Chlor-3-brompropen(1)
trans-1,4-Dibrombuten(2)
1,3-Dichlorpropen(1)
1-Chlor-2-nitro-propan
2-Chlor-1-nitropropan
Trichlornitromethan
Dichlortetrafluoraceton
Natriumsalz der Propionsäure
Calciumsalz der Propionsäure
Chlorfumarsäure-bis-ß-chloräthylester
Sorbinsäure und deren Kaliumsalz
2-Propen-1,1-diolacetat
2-Aminobutan
Dodecylguanidinacetat (dodine)
Dodecylguanidinphthalat
α-Chloracetyl-1,3-aminopropionitril
α-Bromacetylvalinamid
1,2-Dichlor-1-(methylsulfonyl)-äthylen
1,2-Dichlor-1-(butylsulfonyl)-äthylen
trans-1,2-Bis-(n-propylsulfonyl)-äthylen

p-Dichlorbenzol
Hexachlorbenzol (HCB)
1,2,4,5-Tetrachlor-4-nitrobenzol (TECHAZEN)
Pentachlornitrobenzol (QUINTOZEN)
1,3,4-Trichlor-2,4,6-trinitrobenzol
Isomerengemisch von 1,3,4-Trichlor-2,6-dinitrobenzol und 1,2,3-Trichlor-4,6-dinitrobenzol
2,4,5,6-Tetrachlorisophthalsäurenitril
2,4-Dinitrophenyl-thiocyanat
Diphenyl
o-Nitrodiphenyl
1-Chlor-2,4-dinitronaphthalin
Acenaphthen

2,4,6-Trichlorphenol
2,4,5-Trichlorphenol
2,4,5-Trichlorphenylacetat
2,4,5-Trichlorphenyl-chloracetat
Trichlorphenol, Zinksalz
m-Kresylacetat
2,3,4,6-Tetrachlorphenol
Pentachlorphenol (PCP)
o-Dihydroxybenzol
2,4-Dioxy-n-hexylbenzol
2-Phenylphenol
3,5-Dibromsalicylaldehyd
2-Benzyl-4-chlorphenol
2,2'-Dihydroxy-5,5'-dichlor-diphenylmethan (DICHLOROPHEN)
2,2'-Dihydroxy-3,3',5,5', 6,6'-hexachlor-diphenylmethan
2,2'-Dihydroxy-5,5'-dichlor-diphenylsulfid
2,2'-Dihydroxy-3,3',5,5'-tetrachlor-diphenylsulfid
2,2'-Dihydroxy-3,3',5,5'-tetrachlor-diphenylsulfid-di-Natriumsalz
4-Chlor-o-phenylphenol
1,4-Dichlor-2,5-dimethoxybenzol (CHLORONEB).
Salicylanilid
Wismutsalicylat
Mit Chlor oder Brom halogeniertes Trifluormethyl-salicylanilid
Bromiertes Salicylanilid
(3,5-Dimethyl-4-chlorphenoxy)-äthanol
2-(1-Methyl-n-propyl)-4,6-dinitrophenyl-2-methylcrotonat (BINAPACRYL)
2-(1-Methyl-n-propyl)-4,6-dinitrophenylisopropylcarbonat (DINOBUTON)
2-(1-Methyl-n-heptyl)-4,6-dinitrophenylcrotonat (DINOCAP)
Methyl-2,6-dinitro-4-(1-äthyl-hexyl)phenylcarbonat +
Methyl-2,6-dinitro-4-(1-propyl-pentyl)phenylcarbonat (DINOCTON)
4-Nonyl-2,6-dinitro-phenylbutyrat
S-Methyl-2-(1-methyl-n-heptyl)-4,6-dinitrophenylthiocarbonat

2,6-Dichlor-4-nitroanilin (DICHLORAN)
2-Cyanoäthyl-N-phenylcarbamat
Propynyl-N-phenylcarbamat
α-(2-Bromacetoxy)-acetanilid

2,3,5,6-Tetrachlor-benzochinon(1,4) (CHLORANIL)
2,3-Dichlor-naphthochinon (1,4) (DICHLON)
2-Amino-3-chlor-naphthochinon (1,4)
2-Chlor-3-acetamino-naphthochinon(1,4)
4-Methyl-2,3,5,10-tetrahydro-3,5,10-trioxo-4H4-H-naphtho-(1,3-b)-1,4-triazin
2,3,6,7-Tetrachloro-4a-8a-epoxy-1,2,3,4,4a,8a-hexahydro-1,4-methanonaphthalin-5,8-dion
Chinonoximbenzoylhydrazon (BENQUINOX)
N-(Trichlormethylthio)phthalimid (FOLPET)
N-(Trichlormethylthio)cyclohex-4-en-1,2-dicarboximid (CAPTAN)
N-(1,1,2,2-tetrachloräthylthio)cyclohex-4-en-1,2-dicarboximid (CAPTAFOL)
N-Methansulfonyl-N-trichlormethylthio-p-chloranilin
N'-Dichlorfluormethylthio-NN-dimethyl-N'-phenylsulfamid (DICHLORFLUAMID )
S-(2-Pyridyl-1-oxyd)-S'-trichlormethyl-disulfid-Hydrochlorid

O,O,O-Trimethylthiophosphat
O,O-Diäthyl-phthalimidophosphonothioat
5-Amino-bis-(dimethylamido)phosphinyl-3-phenyl-1,2,4-triazol (TRIAMIPHOS)
5-Methylamino-bis-(dimethylamido)phosphinyl-3-phenyl-1,2,4-triazol
O,O-Diäthyl-O-2-pyrazinyl-phosphorthioat
O-Äthyl-S,S-diphenyl-dithiophosphat
O-Äthyl-S-benzyl-phenyldithiophosphonat
O,O-Diäthyl-S-benzyl-thiolphosphat
Zinksalz der Dithiocarbazinsäure
Natrium-N-methyl-dithiocarbamat (METHAM)
Natrium-N-methoxyäthyl-dithiocarbamat

Natrium-N,N-dimethyl-dithiocarbamat (DDC)
Ammonium-N,N-dimethyl-dithiocarbamat
Zink-N,N-dimethyl-dithiocarbamat (ZIRAM)
Eisen-N,N-dimethyl-dithiocarbamat (FERBA)
Kupfer-N,N-dimethyl-dithiocarbamat
Dinatrium-äthylen-1,2-bis-dithiocarbamat (NABAM)
Zink-äthylen-1,2-bis-dithiocarbamat (ZINEB)
Eisen-äthylen-1,2-bis-dithiocarbamat
Mangan(II)-äthylen-1,2-bis-dithiocarbamat (MANEB)
Calcium-äthylen-1,2-bis-dithiocarbamat
Ammonium-äthylen-1,2-bis-dithiocarbamat
Zink-propylen-1,2-bis-dithiocarbamat (MEZINEB) (PROPINEB)
Bis(dimethylthiocarbamoyl)-äthylen-1,2-bis-dithiocarbamat
Komplex bestehend aus (MANEB) und Zinksalz (MANCOZEB)
Tetraäthylthiuram monosulfid

Bis-(N,N-dimethyldithiocarbamylmerkapto)-methylarsin
Tetramethylthiuramdisulfid (THIRAM)
Dipyrrolidylthiuramdisulfid
N,N'-Bis-(dimethylamino)thiuramdisulfid
Polyäthylenthiuramsulfid
Komplex bestehend aus (ZINEB) und polyäthylenthiuramdisulfid (METIRAM)
Bis-(3,4-dichlor-2(5)-furanoyl)äther (mucochloric anhydrid)
2-Methoxymethyl-5-nitrofuran
5-Nitro-furfuraldoxim -(2)
5-Nitro-furfuryl-amidoxim-(2)
1-Oxy-3-acetyl-6-methyl-cyclohexen-(5)-dion-(2,4) (dehydroacetic acid)

3-[-(3,5-Dimethyl-2-oxycyclohexyl)-2-hydroxyäthyl]-glutarimid (cycloheximide)
Phthalimid
Pyridin-2-thiol-1-oxyd-bzw. 1-Hydroxypyridin-2-thion
Zinksalz des Pyridin-2-thiol-1-oxyds
Mangan(II)salz des Pyridin-2-thiol-1-oxyds
S-1(1-Oxido-2-pyridyl)isothiuroniumchlorid

α,α-bis(4-Chlorphenyl)-3-pyridinmethanol (PARINOL)
8-Hydroxychinolin (8-QUINOLINOL)
8-Hydroxychinolin-sulfat (CHINOSOL)
Benzoyl-8-hydroxychinolin-salicylat
3-(2-Methylpiperidino)propyl-3,4-dichlorbenzoat
6-Äthoxy-1,2-dihydro-2,2,4-trimethylchinolin (ETHOXYQUIN)
N-Lauryl-isochinoliniumbromid
9-(p-n-Hexyloxyphenyl)-10-methyl-acridiniumchlorid
9-(p-n-Hexyloxyphenyl)-10-methyl-acridinium-p-toluolsulfonat
2-n-Heptadecylimidazolidinacetat (GLYODIN)
1-Hydroxyäthyl-2-heptadecylimidazolidin
1-Phenyl-3,5-dimethyl-4-nitrosopyrazol
1-p-Chlorphenyl-3,5-dimethyl-4-nitrosopyrazol
1-p-Sulfamylphenyl-3,5-dimethyl-4-nitrosopyrazol
N-(1-Phenyl-2-nitropropyl)piperazin
2-Dimethylamino-6-methyl-5-n-butyl-4-hydroxy-pyrimidin
N-Dodecyl-1,4,5,6-tetrahydropyrimidin
N-Dodecyl-2-methyl-1,4,5,6-tetrahydropyrimidin
2-n-Heptadecyltetrahydropyrimidin
1-(4-Amino-4-propyl-5-pyrimidyl-methyl)-2-methylpyridinium-chloridhydroxychlorid
2-(2'-Furyl)-benzimidazol (FURIDAZOL)
3-Dodecyl-1-methyl-2-phenylbenzimidazolium-ferricyanid
Methyl-N-benzimidazol-2-yl-N-(butylcarbamoyl)carbamat (BENOMYL)
2-(o-Chloranilino)-4,6-dichlor-sym.-triazin
2-Äthylamino-6-methyl-5-n-butyl-4-hydroxy pyrimidin

5-Chlor-4-phenyl-1,2-dithiol-3-on
2,3-Dicyano-1,4-dithia-anthrachinon (DITHIANON)
2-(4-Thiazolyl)-benzimidazol
4-(2-Chlorphenylhydrazono)-3-methyl-5-isoxazolon (DRAZOLON)
Thiazolidinen-4-thion-(2) (RHODANIN)
3-(p-Chlorphenyl)-5-methylrhodanin
3,5-Dimethyltetrahydro-1,3,5-thiadiazin-2-thin (DAZOMET)
3,3'-Äthylen-bis-(tetrahydro-4,6-dimethyl)-2H-1,3,5-thiadazin-2-thion) (MILNEB)
3-Benzylidenamino-4-phenylthiazolin-2-thion

6-Chlorbenzthiazol-2-thiol, Zinksalz
6,ß-Diäthylamino-äthoxy-2-dimethylamino-benzthiazol-dihydrochlorid
Monoäthanolammonium-benzthiazol-2-thiol
Laurylpyridinium-5-chlor-2-merkaptobenzthiazol
Zink- und Natriumsalze des 2-Merkaptobenzthiazols und Dimethyldithiocarbamats
6-(ß-Diäthylaminoäthoxy)-2-dimethylaminobenzthiazol-dihydrochlorid
3-Trichlormethylthiobenzothiazolon
3-Trichlormethylthiobenzoxazolon
3-(Trichlormethyl)-5-äthoxy-1,2,4-thiadiazol
6-Methyl-2-oxo-1,3-dithiolo[4,5-b]-chinoxalin (QUINO-METHIONAT)
2-Thio-1,3-dithiolo[4,5-b]-chinoxalin (THIOQUINOX)
2,3-Dihydro-5-carboxanilido-6-methyl-1,4-oxathin
3,3,4,4-Tetrachlortetrahydrothiophen-1,1-dioxyd
2,3-Dihydro-5-carboxanilido-6-methyl-1,4-oxathin-4,4-dioxyd
Äthyl-trimethylammoniumbromid
n-Alkyl($C_{12}$, $C_{14}$, $C_{16}$)dimethylbenzylammoniumchlorid
Alkenyl-dimethyläthylammoniumbromid
Dialkyldimethylammoniumbromid
Alkyldimethylbenzylammoniumchlorid
Alkyl $C_9$-$C_{15}$ tolylmethyltrimethylammoniumchlorid
Di-isobutylkresoxyäthoxyäthyldimethylbenzylammoniumchlorid
p-Di-isobutylphenoxyäthoxyäthyldimethylbenzylammonium-chlorid
Benzoyltrimethylammoniumbromid

Gliotoxin
2,4-Diguanidino-3,5,6-trihydroxycyclohexyl-5-deoxy-2-O-(2-deoxy-2-methylamino-α-L-glucopyranosyl)3-C-formyl-ß-L-lyxopentofuranosid (STREPTOMYCIN)
7-Chlor-4,6-dimethoxycumaran-3-on-2-spiro-1'-(2'-methoxy-6'-methylcyclohex-2'-en-4'-on) (GRISEOFULVIN)
4-Dimethylamino-1,4,4a,5,5a,6,11,12a-octahydro-3,5,6,10,12,12a-hexahydroxy-6-methyl-1,11-dioxo-2-naphthacen-carboximid (OXYTETRACYCLIN)

7-Chlor-4-dimethylamino-1,4,4a,5,5a,6,11,12a-octahydro-3,6,10,12,12a- pentahydroxy-6-methyl-1,11-dioxo-2-naphthacencarboximid (CHLORTETRACYCLIN)

(PIMARICIN)

(LANCOMYCIN)

(PHLEOMYCIN)

(KASUGAMYCIN)

(PHYTOACTIN)

D(-)-threo-2,2-dichlor-N-[3-hydroxy-α-(hydroxymethyl)-p-nitrophen-äthyl]acetamid (CHLORAMPHENICOL)

Blasticidin-S-benzylamino-benzolsulfonat

N-(3-nitrophenyl)itaconimid

Phenoxyessigsäure

Natrium-p-dimethylamino-benzoldiazosulfonat

Acrolein-phenylhydrazon

2-Chloracetaldehyd(2,4-dinitrophenyl)-hydrazon

2-Chlor-3-(tolylsulfonyl)-propionitril

1-Chlor-2-phenyl-pentan-diol(4,5)-thion(3)

p-Nonylphenoxypolyäthylenoxyäthanol-Jod-Komplex

(α-Nitromethyl)-o-chlorbenzylthioäthylamin-hydrochlorid

3-(p.-t.-butyl-phenylsulfonyl)acrylonitril

Oktachlorcyclohexenon

Pentachlorbenzylalkohol

Pentachlorbenzylacetat

Pentachlorbenzaldehyd-cyanhydrin

2-Norcamphanmethanol

2,6-Bis-(dimethylaminomethyl)-cyclohexanon

Decachloroctahydro-1,3,4-metheno-2H-cyclobuta[cd]-pentalen-2-on

1-(3-Chlorallyl)-3,5,7-triaza -1-azoniaadamantanchlorid

Kohlenteer und Hochofenteer

Mischung Nickelsulfat-Maneb

Mischung Maneb-Merkaptobenzthiazol

Mischung Zineb-Merkaptobenzthiazol

Mischung Zineb-Nickel(II)-chlorid

Mischung Zineb-Nickel(II)-sulfat

Mischung Ziram-basisches Kupfersulfat

Mischung Ziram-Zink-merkaptobenzthiazol
Mischung Thiram-Cadmiumchloridhydrat
Mischung Thiram-Hydroxyquecksilberchlorphenol
Mischung Thiram-Phenylquecksilberacetat
Mischung Polyäthylen-bis-thiuramsulfid-Kupferoxychlorid
Mischung Methylarsin-bis-(dimethyldithiocarbamat)-ziram-thiram
Mischung Folpet-Phenylquecksilberacetat
Mischung Dodine-Ferbam-Schwefel
Mischung Dithianon-Kupferoxychlorid
Mischung Dichlone-Ferbam-Schwefel
Mischung Dinocap-dinitrooctylphenol
Mischung Captan-quintozene-tribasischem Kupfersulfat
Mischung Cadmiumpropionat-Phenylquecksilberpropionat
Formaldehyd-Harnstoff-Mischung
Mischung Phenylammoniumcadmiumdilactat-Phenylquecksilberformamid
Mischung basisches Kupfersulfat-Zinksalze

Die beanspruchten Verbindungen der Formel I eignen sich auch für den Einsatz im technischen Bereich, beispielsweise in Holzschutzmitteln, auf dem Anstrichfarbensektor oder als Konservierungsmittel, z.B. in Kühlschmiermitteln für die Metallbearbeitung.

Die Erfindung wird durch folgende Beispiele näher erläutert:

## A. Herstellungsbeispiele

### Beispiel 1

Methyl-2-/N̄-(2,6-dimethylphenyl)-N-(äthoxythiocarbonylthioacetyl)-amino/-propionat

28,35 g Methyl-2-/N̄-(2,6-dimethylphenyl)-N-(chloracetyl)-amino/-propionat und 15,0 g Natriumäthylxanthogenat werden in 100 ml Aceton gelöst und 24 Stunden bei 30°C gerührt. Anschließend wird vom Ungelösten abfiltriert, mit 300 ml Wasser verdünnt und mit 100 ml Methylenchlorid extrahiert. Die Methylenchloridphase wird abgetrennt, über Natriumsulfat getrocknet und das Methylenchlorid abdestilliert. Man erhält 34,8 g (= 94,5 % d. Theorie) Methyl-2-/N̄-(2,6-dimethylphenyl)-N-(äthoxythiocarbonylthioacetyl)-amino/-propionat als helles Öl mit dem Berechnungsindes $n_D^{22}$ = 1,5629

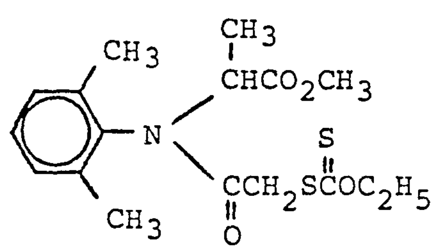

### Beispiel 2

Methyl-2-/N̄-(2,6-dimethylphenyl)-N-(methoxythiocarbonylthioacetyl)-amino/-propionat

28,1 g Methyl-2-/N̄-(2,6-dimethylphenyl)-(merkaptoacetyl)-amino/-propionat (s. Beisp. 9) und 11,1 g Triäthylamin werden in 100 ml Toluol gelöst und bei 0°C mit 12,1 g O-Methyl-thiokohlensäurechlorid versetzt. Anschließend wird 2 h bei Raumtemperatur und 1 h bei 40°C gerührt. Der Niederschlag von TÄA-Hydrochlorid wird abfiltriert und die Toluolphase mit Wasser gewaschen, über Natriumsulfat getrocknet und anschließend wird Toluol unter Wasserstrahlvakuum abdestilliert. Man erhält 31,9 g

(= 90 % d. Theorie) Methyl-2-/N-(2,6-dimethylphenyl)-N-(methoxythiocarbonylthioacetyl)-amino/-propionat als gelbes Öl mit dem Brechungsindex $n_D^{22}$ = 1,5681.

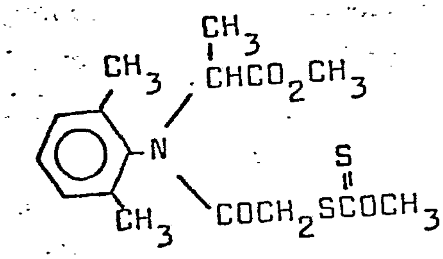

Beispiel 3

2-[N-(2,6-Dimethylphenyl)-äthoxythiocarbonylthioacetyl-amino]-butyrolacton

28,15 g 2-[N-(2,6-Dimethylphenyl)-chloracetylamino]-butyrolacton und 15,2 g Natriumäthylxanthogenat werden in 100 ml Aceton gelöst und 2 Tage bei 30°C belassen. Der Salzniederschlag wird abfiltriert, das Filtrat mit 200 ml Wasser verdünnt und mit Methylenchlorid extrahiert. Die Methylenchloridphase wird über Natriumsulfat getrocknet und das Methylenchlorid abdestilliert. Man erhält 34,1 g (= 93 % d.Theorie) 2-[N-(2,6-Dimethylphenyl)-äthoxythiocarbonylthioacetylamino]-butyrolacton als zähes, gelbes Öl.

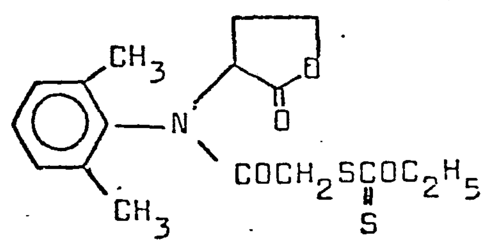

Beispiel 4 AD

D-Methyl-2-[N-(äthoxycarbonylthioacetyl)-N-(2,6-dimethylphenyl)-amino]-propionat

12,4 g (0,06 Mol) D-Methyl-2-[N-(2,6-dimethylphenyl)-amino]-propionat werden in 60 ml Toluol bei 90° - 100°C mit 11,5 g (0,063 Mol) Äthoxycarbonylthioacetylchlorid versetzt und bis zur Beendigung der Gasentwicklung bei 100°C gerührt. Nach dem Abkühlen wird mit Natriumbikarbonatlösung neutralisiert, die Toluolphase abgetrennt, über Natriumsulfat getrocknet und Toluol abdestilliert. Nach dem Umkristallisieren aus Toluol/Cyclohexan erhält man 18,9 g (85 % d.Th.) D-Methyl-2[N-(2,6-dimethylphenyl)-N-(äthoxycarbonylthioacetyl)-amino]-propionat vom Schmelzpunkt 77°C und einer optischen Drehung von $[\alpha_D]^{24°C} = -28,7$ °.

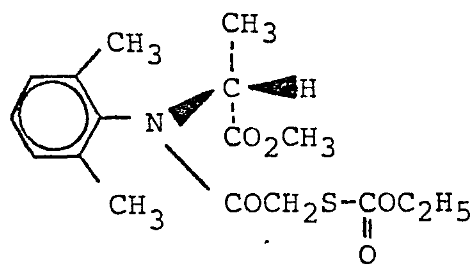

Beispiel 5

Methyl-2-[N-(5-aza-3-thia-5-cyan-3-methylthio-4-penteno-1-yl)-N-(2,6-dimethylphenyl)amino]-propionat

28,35 g (0,1 Mol) Methyl-2-[N-(chloracetyl)-N-(2,6-dimethylphenyl)amino]-propionat und 18,4 g (0,11 Mol) Kalium-cyanimidothiokohlensäure-S-monomethylester werden in 100 ml Acetonitril 12 Stunden bei 60°C gerührt. Nach Abfiltrieren des entstandenen Salzniederschlags wird das Filtrat mit Eis und Wasser verdünnt, der gebildete Feststoff abfiltriert und aus Toluol umkristallisiert. Man erhält 33 g (87 % d.Th.) Methyl-2[N-(5-aza-3-thia-5-cyan-3-methylthio-4-penteno-1-yl)-N-(2,6-dimethylphenyl)-amino]-propionat vom Schmelzpunkt 109 - 111°C.

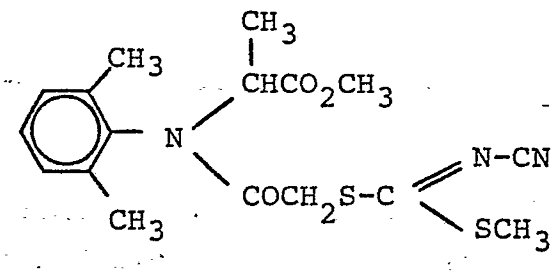

Beispiel 6

Methyl-2-/N̄-(methylaminothiocarbonylthioacetyl)-N-(2,6-dimethylphenyl)-amino̲7-propionat

Zu einer Lösung von 18,56 g (0,6 Mol) Methylamin in 300 ml Methanol gibt man bei Raumtemperatur zunächst 22,8 g (0,3 Mol) Schwefelkohlenstoff, dann 85,05 g (0,3 Mol) Methyl-2-/N̄-(chloracetyl)-N-(2,6-dimethylphenyl)-amino̲7-propionat und erhitzt anschließend 8 Stunden auf 50 - 60°C. Der Ansatz wird mit Eis und Wasser verdünnt und der entstandene Niederschlag abfiltriert und getrocknet. Man erhält 94,5 g (89 % d.Th.) Methyl-2-/N̄-(methylaminothiocarbonylthioacetyl)-N-(2,6-dimethylphenyl)-amino̲7-propionat mit dem Schmelzpunkt 119 - 121°C.

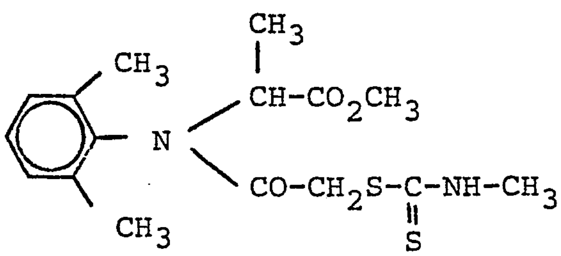

Beispiel 7

Methyl-2-/N̄-(5-aza-3-thia-4-äthylthio-hex-4-eno-1-yl)-N-(2,6-dimethylphenyl)-amino̲7-propionat

17,7 g (0,05 Mol) Methyl-2-/N̄-(methylaminothiocarbonylthioacetyl)-N-(2,6-dimethylphenyl)-amino̲7-propionat, 9,6 g (0,06 Mol) Äthyljodid, 6,9 g (0,05 Mol) Kaliumcarbonat werden in 60 ml Methanol 16 Stunden bei 55 - 60°C gerührt. Nach Abfiltrieren des Salzniederschlags wird

mit Eis und Wasser verdünnt und mit Methylenchlorid extrahiert. Die Methylenchloridphase wird über Natriumsulfat getrocknet, Methylenchlorid wird abdestilliert und der halbfeste Rückstand wird aus Isopropanol/Wasser umkristallisiert. Man erhält 13,2 g (69 % d.Th.) Methyl-2-/N̄-(5-aza-3-thia-4-äthylthio-hex-4-eno-1-yl)-N-(2,6-dimethylphenyl)-amino/-propionat mit dem Schmelzpunkt 57,5 - 58,5°C.

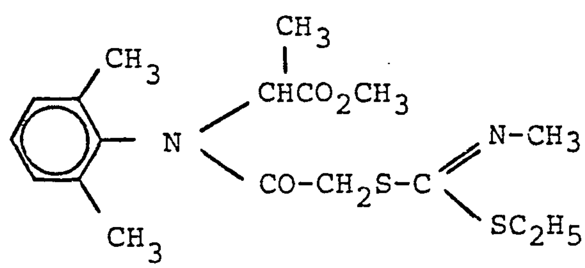

Beispiel 8

Methyl-2-/N̄-(acetylthioacetyl)-N-(2,6-dimethylphenyl)-amino/-propionat

28,35 g (0,1 Mol) Methyl-2-/N̄-(chloracetyl)-N-(2,6-dimethylphenyl)-amino/-propionat und 9,1 g (0,12 Mol) Thioessigsäure in 100 ml Acetonitril werden bei Eiskühlung mit 12,1 g (0,12 Mol) Triäthylamin versetzt, anschließend wird 3 Stunden bei 50°C nachgerührt. Nach üblicher Aufarbeitung wird durch Destillation gereinigt. Man erhält 24,2 g (74,8 % d.Th.) Methyl-2-/N̄-(acetylthioacetyl)-N-(2,6-dimethylphenyl)-amino/-propionat mit dem Siedepunkt 175 - 178°C bei 0,025 mb.

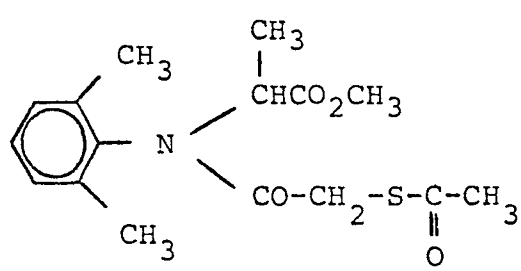

Beispiel 9

Methyl-2-/N-(merkaptoacetyl)-N-(2,6-dimethylphenyl)-amino/-propionat

25 g (0,0445 Mol) Dithiodiglykolsäure-bis-(N-1-methoxycarbonyläthyl)-2,6-dimethylanilid (erhalten durch Umsetzung von Methyl-2-(2,6-dimethylphenylamino)-propionat und Dithiodiglykolsäuredichlorid), 85 ml Wasser, 42,5 ml konz. Salzsäure und 200 ml Methanol werden vorgelegt und mit 20 g Zinkstaub portionsweise bei 0 - 10°C versetzt. Anschließend wird 3 Stunden nachgerührt, mit Eiswasser verdünnt und mit Methylenchlorid extrahiert. Nach Trocknen und Abdestillieren von Methylenchlorid erhält man 25 g (99,2 % d.Th.) praktisch reines Methyl-2-/N-(merkaptoacetyl)-N-(2,6-dimethylphenyl)-amino/-propionat mit dem Schmelzpunkt 62,5 - 63°C.

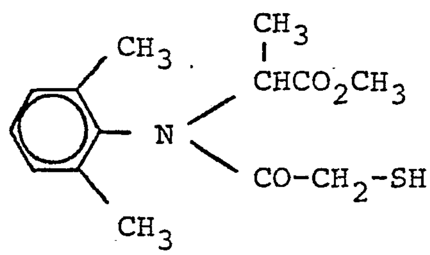

Beispiele 10 bis 152

In den nachfolgenden Tabellen 1 bis 3 sind die Beispiele 10 bis 152 für Verbindungen der Formel I aufgeführt, und zwar in Tabelle 1 die Beispiele 10 bis 65, in Tabelle 2 die Beispiele 66 bis 90 und in Tabelle 3 die Beispiele 91 bis 152, wobei neben den einzelnen Substituenten in Formel I und den physikalisch-chemischen Kenndaten der Produkte jeweils in der letzten Spalte der Tabellen auch die Art der Herstellung der Verbindung der Formel I analog einem der vorgenannten Beispiele 1 bis 9 angegeben ist.

In Tabelle 4 ist zusätzlich eine Auwahl von weiteren 14 Beispielen mit optisch aktiven Verbindungen der Formel I der D-Reihe aufgeführt. Diese Produkte werden von den Beispielen mit den entsprechenden racemischen Produkten durch den Zusatz "AD" zur Beispiel-Nr. unterschieden. In der Tabelle 4 werden diese optisch aktiven Verbindungen neben den entsprechenden Substituenten in Formel I, der Herstellungsart und den üblichen physikalisch-chemischen Kenndaten außerdem durch ihre spezifische Drehung $[\alpha]^t_D$ charakterisiert.

Tabelle 1

Formel I mit X = 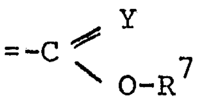 : 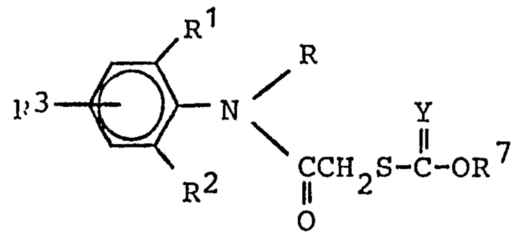

| Beispiel Nr. | $R^1$ | $R^2$ | $R^3$ | R | Y | $R^7$ | Fp./Kp. $n_D$ | Herstellung analog Beispiel Nr. |
|---|---|---|---|---|---|---|---|---|
| 10 | $-CH_3$ | $-CH_3$ | -H | $-CH(CH_3)CO_2CH_3$ | S | $-i-C_3H_7$ | $n_D^{22} = 1,5529$ | 1 |
| 11 | $-CH_3$ | $-CH_3$ | -H | " | S | $-n-C_3H_7$ | $n_D^{20} = 1,5570$ | 1 |
| 12 | $-CH_3$ | $-CH_3$ | -H | " | S | $-s-C_4H_9$ | $n_D^{20} = 1,5542$ | 1 |
| 13 | $-CH_3$ | $-CH_3$ | -H | " | S | $-i-C_4H_9$ | $n_D^{24} = 1,5505$ | |
| 14 | $-CH_3$ | $-CH_3$ | -H | " | S | $-n-C_5H_9$ | | 1 |
| 15 | $-CH_3$ | $-CH_3$ | -H | " | S | 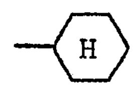 | $n_D^{15} = 1,5669$ | 1 |
| 16 | $-CH_3$ | $-CH_3$ | -H | " | S | $-CH_2CH_2OCH_3$ | Fp.: 60 - 62°C | 1 |
| 17 | $-CH_3$ | $-CH_3$ | -H | " | S | $-CH_2CH=CH_2$ | | 1 |
| 18 | $-CH_3$ | $-CH_3$ | -H | " | S | $-CH_2C\equiv CH$ | | 1 |

(Fortsetzung von Tab. 1)

| Beispiel-Nr. | $R^1$ | $R^2$ | $R^3$ | R | y | $R^7$ | Fp./Kp./$n_D$ | Herstellung analog Beisp.-Nr. |
|---|---|---|---|---|---|---|---|---|
| 19 | $-CH_3$ | $-CH_3$ | -H | $-\overset{CH_3}{\overset{\vert}{C}}HCO_2CH_3$ | O | $-C_2H_5$ | Fp.: 76 - 78°C | 2 |
| 20 | $-CH_3$ | $-CH_3$ | -H | " | O | $-CH_3$ | Fp.: 77,5 - 79°C | 4 AD |
| 21 | $-CH_3$ | $-C_2H_5$ | -H | " | S | $-C_2H_5$ | $n_D^{17}$ = 1,5612 | 1 |
| 22 | $-C_2H_5$ | $-C_2H_5$ | -H | " | S | $-C_2H_5$ | $n_D^{20}$ = 1,5600 | 1 |
| 23 | $-i-C_3H_7$ | H | -H | " | S | $-C_2H_5$ | Fp.: 83°C | 1 |
| 24 | $-i-C_3H_7$ | H | -H | " | S | $-i-C_3H_7$ | $n_D^{18}$ = 1,5516 | 1 |
| 25 | $-CH_3$ | $-CH_3$ | $3-CH_3$ | " | S | $-C_2H_5$ | | 1 |
| 26 | $-CH_3$ | -H | -H | $-CH_2CO_2CH_3$ | S | $-C_2H_5$ | $n_D^{24}$ = 1,5665 | 1 |
| 27 | $-i-C_3H_7$ | -H | -H | " | S | $-C_2H_5$ | $n_D^{22}$ = 1,5602 | 1 |
| 28 | $-CH_3$ | -H | $4-CH_3$ | " | S | $-C_2H_5$ | $n_D^{19}$ = 1,5600 | 1 |
| 29 | $-CH_3$ | -H | $3-CH_3$ | " | S | $-C_2H_5$ | $n_D^{23,5}$ = 1,5670 | 1 |
| 30 | $-CH_3$ | $-CH_3$ | -H | " | S | $-C_2H_5$ | $n_D^{24}$ = 1,5681 | 1 |
| 31 | $-CH_3$ | $-CH_3$ | -H | " | O | $-CH_3$ | $n_D^{24}$ = 1,5392 | 4 AD |
| 32 | $-CH_3$ | $-CH_3$ | -H | " | O | $-C_2H_5$ | | 4 AD |

0023332

(Fortsetzung von Tab. 1)

| Beispiel Nr. | $R^1$ | $R^2$ | $R^3$ | R | Y | $R^7$ | Fp./Kp./$n_D$ | Herstellung analog Beisp.-Nr. |
|---|---|---|---|---|---|---|---|---|
| 33 | $-CH_3$ | $-CH_3$ | -H | $-CH_2CO_2C_2H_5$ | S | $-C_2H_5$ | $n_D^{22} = 1{,}5608$ | 1 |
| 34 | -Cl | $-CH_3$ | -H | $-CH_2CO_2CH_3$ | S | $-C_2H_5$ | $n_D^{17} = 1{,}5796$ | 1 |
| 35 | $-CH_3$ | $-CH_3$ | -H | 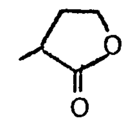 | S | $-CH_3$ | | 3 |
| 36 | $-CH_3$ | $-CH_3$ | -H | 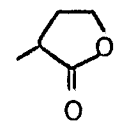 | S | $-i-C_3H_7$ | Fp.: 95 - 96°C | 3 |
| 37 | $-CH_3$ | $-CH_3$ | -H | $CH_3$<br>$-CHCO_2C_2H_5$ | S | $-C_2H_5$ | $n_D^{23,5} = 1{,}5516$ | 1 |
| 38 | $-CH_3$ | -Cl | -H | $CH_3$<br>$-CHCO_2CH_3$ | S | $-n-C_3H_7$ | $n_D^{21} = 1{,}5648$ | 1 |
| 39 | $-CH_3$ | -Cl | -H | $CH_3$<br>$-CHCO_2CH_3$ | S | $-i-C_3H_7$ | $n_D^{21} = 1{,}5644$ | 1 |
| 40 | $-CH_3$ | $-CH_3$ | -H | $CH_3$<br>$-CHCOSCH_3$ | S | $-C_2H_5$ | | 1 |

(Fortsetzung von Tab. 1)

| Beispiel Nr. | $R^1$ | $R^2$ | $R^3$ | R | Y | $R^7$ | Fp./Kp./$n_D$ | Herstellung analog Beisp.-Nr. |
|---|---|---|---|---|---|---|---|---|
| 41 | $-CH_3$ | $-CH_3$ | -H | $CH_3$<br>$-CHCO-SCH_3$ | S | $-CH_3$ | | 2 |
| 42 | $-CH_3$ | -Cl | -H | $CH_3$<br>$-CHCO_2CH_3$ | O | $-CH_3$ | $n_D^{24} = 1,5413$ | 4 AD |
| 43 | $-CH_3$ | $-CH_3$ | -H | $CH_3$<br>$-CHCO_2N(CH_3)_2$ | S | $-C_2H_5$ | | 1 |
| 44 | $-C_2H_5$ | $-C_2H_5$ | -H | $-CH_2CO_2C_2H_5$ | S | $-C_2H_5$ | | 1 |
| 45 | $-CH_3$ | $-C_2H_5$ | -H | $CH_3$<br>$-CHCO_2CH_3$ | S | $-n-C_3H_7$ | $n_D^{21} = 1,5568$ | 1 |
| 46 | $-CH_3$ | $-C_2H_5$ | -H | $CH_3$<br>$-CHCO_2CH_3$ | S | $-i-C_3H_7$ | $n_D^{20} = 1,5569$ | 1 |
| 47 | $-CH_3$ | $-CH_3$ | -H | $CH_3$<br>$-CHCO_2C_2H_5$ | S | $-i-C_3H_7$ | $n_D^{23,5} = 1,5495$ | 1 |
| 48 | $-CH_3$ | -Cl | -H | $CH_3$<br>$-CHCO_2CH_3$ | S | $-C_2H_5$ | $n_D^{17} = 1,5796$ | 1 |
| 49 | $-CH_3$ | -Cl | -H | $CH_3$<br>$-CHCO_2CH_3$ | S | $-CH_3$ | | 2 |

(Fortsetzung von Tab. 1)

| Beispiel Nr. | $R^1$ | $R^2$ | $R^3$ | R | Y | $R^7$ | Fp-/Kp./$n_D$ | Herstellung analog Beisp.-Nr. |
|---|---|---|---|---|---|---|---|---|
| 50 | $-CH_3$ | $-C_2H_5$ | -H | $-\overset{CH_3}{\overset{\vert}{C}}HCO_2CH_3$ | O | $-C_2H_5$ | Fp.: 73 - 76°C | 4 AD |
| 51 | $-CH_3$ | $-C_2H_5$ | -H | " | O | $-CH_3$ | Fp.: 86 - 89°C | 4 AD |
| 52 | $-CH_3$ | -Cl | -H | $-CH_2CO_2CH_3$ | O | $-C_2H_5$ | $n_D^{24}$ = 1,5387 | 4 AD |
| 53 | $-CH_3$ | $-CH_3$ | -H | $-\overset{CH_3}{\overset{\vert}{C}}HCO_2C_2H_5$ | O | $-CH_3$ | | 4 AD |
| 54 | $-CH_3$ | -H | -H | $-\overset{CH_3}{\overset{\vert}{C}}HCO_2CH_3$ | O | $-CH_3$ | $n_D^{20}$ = 1,5329 | 4 AD |
| 55 | $-CH_3$ | -H | $3-CH_3$ | " | O | $-CH_3$ | $n_D^{20}$ = 1,5350 | 4 AD |
| 56 | $-C_2H_5$ | $-C_2H_5$ | -H | " | O | $-CH_3$ | $n_D^{20}$ = 1,5313 | 4 AD |
| 57 | $-CH_3$ | $-CH_3$ | -H | 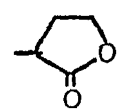 | O | $-CH_3$ | | 4 AD |
| 58 | $-CH_3$ | $-CH_3$ | -H | 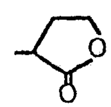 | O | $-C_2H_5$ | | 4 AD |
| 59 | $-CH_3$ | $-CH_3$ | -H | 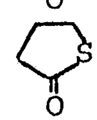 | O | $-CH_3$ | | 4 AD |
| 60 | $-CH_3$ | $-CH_3$ | -H | " | S | $-C_2H_5$ | | 1 |
| 61 | $-CH_3$ | $-CH_3$ | -H | " | S | $-CH_3$ | | 2 |

(Fortsetzung von Tab. 1)

| Beispiel Nr. | $R^1$ | $R^2$ | $R^3$ | R | Y | $R^7$ | Fp./Kp./$n_D$ | Herstellung analog Beisp.-Nr. |
|---|---|---|---|---|---|---|---|---|
| 62 | $-CH_3$ | $-CH_3$ | -H | $CH_3$<br>$-CHCO_2CH_3$ | O | $-i-C_3H_7$ | | 4 AD |
| 63 | $-CH_3$ | $-CH_3$ | -H | " | O | $-n-C_3H_7$ | | 4 AD |
| 64 | $-CH_3$ | $-CH_3$ | -H | " | O | $-s-C_4H_9$ | | 4 AD |
| 65 | $-CH_3$ | $-CH_3$ | -H | " | O | $-CH_2CH_2OCH_3$ | | 4 AD |

<u>Tabelle 2</u>

Formel I mit X = 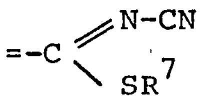 : 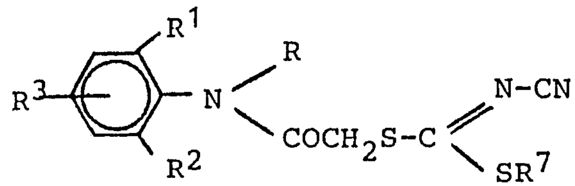

| Beispiel Nr. | $R^1$ | $R^2$ | $R^3$ | R | $R^7$ | physik.-chem. Kenndaten | Herstellung analog Beisp.-Nr. |
|---|---|---|---|---|---|---|---|
| 66 | $-CH_3$ | $-CH_3$ | -H | 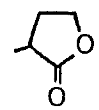 | $-CH_3$ | | 5 |
| 67 | $-CH_3$ | $-CH_3$ | -H | 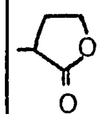 | $-C_2H_5$ | | 5 |
| 68 | $-CH_3$ | $-CH_3$ | -H | $-CH_2CO_2CH_3$ | $-CH_3$ | Fp.: 90°C | 5 |
| 69 | $-CH_3$ | -H | $3-CH_3$ | $-CH_2CO_2CH_3$ | $-CH_3$ | zähes Öl | 5 |
| 70 | $-CH_3$ | $-CH_3$ | -H | $-CH_2CO_2CH_3$ | $-C_2H_5$ | | 5 |
| 71 | $-CH_3$ | $-CH_3$ | -H | $-CH(CH_3)CO_2CH_3$ | $-C_2H_5$ | Fp.: 106 - 107°C | 5 |
| 72 | $-CH_3$ | $-CH_3$ | -H | $-CH(CH_3)CO_2CH_3$ | $-n-C_3H_7$ | | 5 |
| 73 | $-CH_3$ | $-CH_3$ | -H | $-CH(CH_3)CO_2CH_3$ | $-i-C_3H_7$ | | 5 |
| 74 | $-CH_3$ | $-CH_3$ | -H | $-CH(CH_3)CO_2CH_3$ | $-CH_2CH=CH_2$ | | 5 |
| 75 | $-CH_3$ | $-CH_3$ | -H | $-CH(CH_3)CO_2CH_3$ | $-CH_2C\equiv CH$ | | 5 |

(Fortsetzung von Tabelle 2)

| Beispiel Nr. | $R^1$ | $R^2$ | $R^3$ | R | $R^7$ | physik.-chem. Kenndaten | Herstellung analog Beisp.-Nr. |
|---|---|---|---|---|---|---|---|
| 76 | $-CH_3$ | $-CH_3$ | -H | $-\overset{CH_3}{\overset{\vert}{C}}HCO_2CH_3$ | 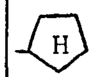 | | 5 |
| 77 | $-CH_3$ | $-CH_3$ | -H | $-\overset{CH_3}{\overset{\vert}{C}}HCO_2C_2H_5$ | $-CH_3$ | Fp.: 97 - 99°C | 5 |
| 78 | $-CH_3$ | $-CH_3$ | -H | 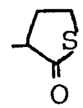 | $-CH_3$ | | 5 |
| 79 | $-CH_3$ | $-CH_3$ | -H | 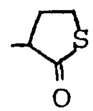 | $-C_2H_5$ | | 5 |
| 80 | $-CH_3$ | $-CH_3$ | -H | $-\overset{CH_3}{\overset{\vert}{C}}HCOSCH_3$ | $-CH_3$ | | 5 |
| 81 | $-CH_3$ | -Cl | -H | $-CH_2CO_2CH_3$ | $-CH_3$ | | 5 |
| 82 | $-CH_3$ | -Cl | -H | $-CH_2CO_2CH_3$ | $-C_2H_5$ | | 5 |
| 83 | $-C_2H_5$ | $-CH_3$ | -H | $-CH_2CO_2CH_3$ | $-CH_3$ | | 5 |
| 84 | $-CH_3$ | -Cl | -H | $-\overset{CH_3}{\overset{\vert}{C}}HCO_2CH_3$ | $-CH_3$ | zähes Öl | 5 |
| 85 | $-CH_3$ | -Cl | -H | $-\overset{CH_3}{\overset{\vert}{C}}HCO_2CH_3$ | $-C_2H_5$ | | 5 |
| 86 | $-C_2H_5$ | $-CH_3$ | -H | $-\overset{CH_3}{\overset{\vert}{C}}HCO_2CH_3$ | $-CH_3$ | zähes Öl | 5 |
| 87 | $-C_2H_5$ | $-CH_3$ | -H | $-\overset{CH_3}{\overset{\vert}{C}}HCO_2CH_3$ | $-C_2H_5$ | | 5 |

(Fortsetzg.v. Tab. 2)

| Beispiel Nr. | $R^1$ | $R^2$ | $R^3$ | R | $R^7$ | physik.-chem. Kenndaten | Herstellung analog Beisp.-Nr. |
|---|---|---|---|---|---|---|---|
| 88 | $-CH(CH_3)_2$ | -H | -H | $CH_3$<br>$-CHCO_2CH_3$ | $-CH_3$ | $n_D^{22}$ = 1,5710 | 5 |
| 89 | $-CH_3$ | -H | -H | $-CH_2CO_2CH_3$ | $-CH_3$ | zähes Öl | 5 |
| 90 | $-CH_3$ | $-CH_3$ | -H | $CH_3$<br>$-CHCO_2CH(CH_3)_2$ | $-CH_3$ | $n_D^{19}$ = 1,5590 | 5 |

Tabelle 3

Formel I: 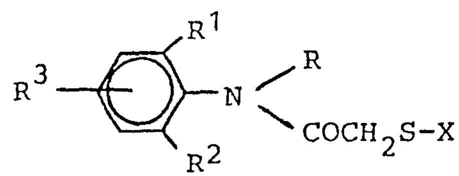

| Beispiel Nr. | $R^1$ | $R^2$ | $R^3$ | R | X | physik.-chem. Kenndaten | Herstellung analog Beisp.-Nr. |
|---|---|---|---|---|---|---|---|
| 91 | $-CH_3$ | $-CH_3$ | -H | $-\overset{CH_3}{CH}-CO_2CH_3$ | $-\overset{S}{C}-S-C_2H_5$ | $n_D^{20}$ = 1,5612 | 1 |
| 92 | $-CH_3$ | $-CH_3$ | -H | " | $-\overset{S}{C}-SCH_3$ | | 1 |
| 93 | $-CH_3$ | -Cl | -H | " | $-\overset{S}{C}-SC_2H_5$ | | 1 |
| 94 | $-CH_3$ | $-CH_3$ | -H | " | $-\overset{O}{C}-SC(CH_3)_3$ | | 2 |
| 95 | $-CH_3$ | $-CH_3$ | -H | $-CH_2CO_2CH_3$ | $-\overset{S}{C}-SC_2H_5$ | | 1 |
| 96 | $-CH_3$ | $-CH_3$ | -H | 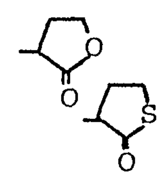 | $-\overset{S}{C}-SC_2H_5$ | $n_D^{20}$ = 1,6086 | 1 |
| 97 | $-CH_3$ | $-CH_3$ | -H | | $-\overset{S}{C}SC_2H_5$ | | 1 |
| 98 | $-CH_3$ | $-CH_3$ | -H | $-\overset{CH_3}{CH}CO_2CH_3$ | $-\overset{O}{C}SC_2H_5$ | | 2 |
| 99 | $-CH_3$ | $-CH_3$ | -H | " | $-\overset{O}{C}-SCH_3$ | | 2 |
| 100 | $-CH_3$ | $-CH_3$ | -H | 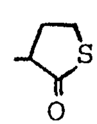 | $-\overset{O}{C}-SC_2H_5$ | | 4 AD |

(Fortsetzung von Tab. 3)

| Beispiel Nr. | $R^1$ | $R^2$ | $R^3$ | R | X | physik.-chem- Kenndaten | Herstellung analog Beisp.-Nr. |
|---|---|---|---|---|---|---|---|
| 101 | $-CH_3$ | $-CH_3$ | -H | $-\overset{\overset{CH_3}{\mid}}{C}HCO_2CH_3$ | $-\overset{\overset{O}{\|}}{C}-NH-CH_3$ | Fp.: 114 - 115°C | 2 |
| 102 | $-CH_3$ | $-CH_3$ | -H | " | $-\overset{\overset{O}{\|}}{C}-NH-C_2H_5$ | Fp.: 141 - 142,5°C | 2 |
| 103 | $-CH_3$ | $-CH_3$ | -H | " | $-\overset{\overset{O}{\|}}{C}-NH-C_4H_9(n)$ | Fp.: 99 - 100°C | 2 |
| 104 | $-CH_3$ | $-CH_3$ | -H | " | $-\overset{\overset{O}{\|}}{C}-N(CH_3)_2$ | Fp.: 124,5 - 125,5°C | 2 |
| 105 | $-CH_3$ | $-CH_3$ | -H | " | $-\overset{\overset{O}{\|}}{C}-N(C_2H_5)_2$ | $n_D^{20}$ = 1,5336 | 2 |
| 106 | $-CH_3$ | $-CH_3$ | -H | 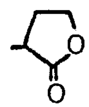 | $-\overset{\overset{O}{\|}}{C}-NH-CH_3$ | | 2 |
| 107 | $-CH_3$ | $-CH_3$ | -H | 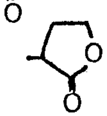 | $-\overset{\overset{O}{\|}}{C}-N(CH_3)_2$ | | 2 |
| 108 | $-CH_3$ | $-CH_3$ | -H | 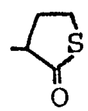 | $-\overset{\overset{O}{\|}}{C}-NH-CH_3$ | | 2 |
| 109 | $-CH_3$ | $-CH_3$ | -H | " | $-\overset{\overset{O}{\|}}{C}-N(CH_3)_2$ | | 2 |
| 110 | $-CH_3$ | $-CH_3$ | -H | 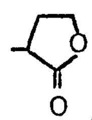 | $-\overset{\overset{S}{\|}}{C}-NH-CH_3$ | | 6 |
| 111 | $-CH_3$ | $-CH_3$ | -H | 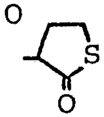 | $-\overset{\overset{S}{\|}}{C}-NH-CH_3$ | | 6 |

(Fortsetzung von Tab. 3)

| Beispiel Nr. | $R^1$ | $R^2$ | $R^3$ | R | X | physik.-chem-Kenndaten | Herstellung analog Beisp.-Nr. |
|---|---|---|---|---|---|---|---|
| 112 | $-CH_3$ | $-CH_3$ | -H | $-\overset{CH_3}{\overset{\vert}{C}}H-COSCH_3$ | $-\overset{O}{\overset{\|}{C}}-NH-CH_3$ | | 2 |
| 113 | $-CH_3$ | $-CH_3$ | -H | " | $-\overset{S}{\overset{\|}{C}}-NH-CH_3$ | | 6 |
| 114 | $-CH_3$ | $-CH_3$ | -H | " | $-\overset{O}{\overset{\|}{C}}-N(CH_3)_2$ | | 2 |
| 115 | $-CH_3$ | $-CH_3$ | -H | $-\overset{CH_3}{\overset{\vert}{C}}H-CO_2CH_3$ | $-\overset{S}{\overset{\|}{C}}-NH-C_2H_5$ | Fp.: 113 - 114°C | 6 |
| 116 | $-CH_3$ | $-CH_3$ | -H | " | $-\overset{S}{\overset{\|}{C}}-NH-C_3H_7(i)$ | Fp.: 107,5 - 108,5°C | 6 |
| 117 | $-CH_3$ | $-CH_3$ | -H | " | $-\overset{S}{\overset{\|}{C}}-NH-C_4H_9(n)$ | Fp.: 104 - 105°C | 6 |
| 118 | $-CH_3$ | $-CH_3$ | -H | " | $-\overset{S}{\overset{\|}{C}}-NH-C_3H_7(n)$ | Fp.: 120 - 120,5°C | 6 |
| 119 | $-CH_3$ | $-CH_3$ | -H | " | $-\overset{S}{\overset{\|}{C}}-NH-C_4H_9(i)$ | Fp.: 126 - 127°C | 6 |
| 120 | $-CH_3$ | $-CH_3$ | -H | " | $-\overset{S}{\overset{\|}{C}}-NH-C_4H_9(t)$ | $n_D^{25,5}$ = 1,5465 | 6 |
| 121 | $-CH_3$ | $-CH_3$ | -H | " | $-\overset{S}{\overset{\|}{C}}-NH-CH_2-$ 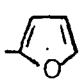 | Fp.: 117 - 119°C | 6 |
| 122 | $-CH_3$ | $-CH_3$ | -H | " | $-\overset{S}{\overset{\|}{C}}-NH-CH_2CH_2OCH_3$ | Fp.: 80 - 81°C | 6 |
| 123 | $-CH_3$ | $-CH_3$ | -H | " | $-\overset{S}{\overset{\|}{C}}-NH-CH_2CH=CH_2$ | Fp.: 116 - 118°C | 6 |

(Fortsetzung von Tab. 3)

| Beispiel Nr. | $R^1$ | $R^2$ | $R^3$ | R | X | physik.-chem. Kenndaten | Herstellung analog Beisp.-Nr. |
|---|---|---|---|---|---|---|---|
| 124 | $-CH_3$ | $-CH_3$ | -H | 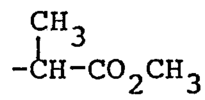 | 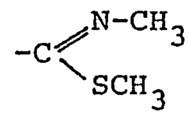 | $n_D^{20}$ = 1,5412 | 7 |
| 125 | $-CH_3$ | $-CH_3$ | -H | 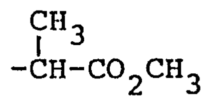 | 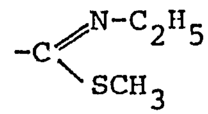 | $n_D^{26}$ = 1,5349 | 7 |
| 126 | $-CH_3$ | $-CH_3$ | -H | " | 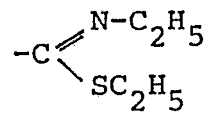 | $n_D^{26}$ = 1,5363 | 7 |
| 127 | $-CH_3$ | $-CH_3$ | -H | " | 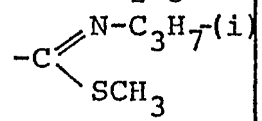 | | 7 |
| 128 | $-CH_3$ | $-CH_3$ | -H | " | 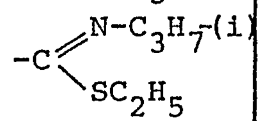 | $n_D^{26}$ = 1,5286 | 7 |
| 129 | $-CH_3$ | $-CH_3$ | -H | " | 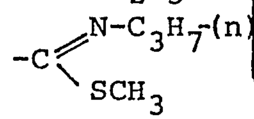 | $n_D^{26}$ = 1,5256 | 7 |
| 130 | $-CH_3$ | $-CH_3$ | -H | " | 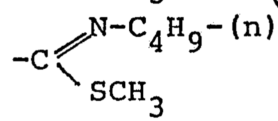 | $n_D^{20}$ = 1,5528 | 7 |
| 131 | $-CH_3$ | $-CH_3$ | -H | 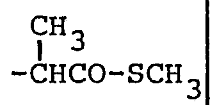 | 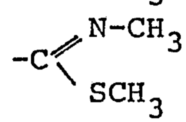 | | 7 |
| 132 | $-CH_3$ | $-CH_3$ | -H | 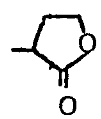 | " | | 7 |

(Fortsetzung von Tab. 3)

| Beispiel Nr. | $R^1$ | $R^2$ | $R^3$ | R | X | physik.-chem. Kenndaten | Herstellung analog Beisp.-Nr. |
|---|---|---|---|---|---|---|---|
| 133 | $-CH_3$ | $-CH_3$ | -H | 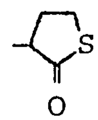 | $-C(=N-CH_3)SCH_3$ | | 7 |
| 134 | $-CH_3$ | $-C_2H_5$ | -H | $-CH(CH_3)-CO_2CH_3$ | $-C(=S)NH-CH_3$ | $n_D^{22,5} = 1,5454$ | 6 |
| 135 | $-C_3H_7(i)$ | -H | -H | " | " | Fp.: 125 - 127°C | 6 |
| 136 | $-C_2H_5$ | $-C_2H_5$ | -H | $-CH_2CO_2C_2H_5$ | " | Fp.: 67 - 69°C | 6 |
| 137 | $-CH_3$ | $-CH_3$ | -H | $-CH_2-CO_2CH_3$ | $-C(=O)-NH-CH_3$ | | 2 |
| 138 | $-CH_3$ | $-CH_3$ | -H | " | $-C(=O)-N(CH_3)_2$ | | 2 |
| 139 | $-CH_3$ | $-CH_3$ | -H | " | $-C(=S)-NH-CH_3$ | | 6 |
| 140 | $-CH_3$ | $-CH_3$ | -H | " | $-C(=N-CH_3)SCH_3$ | | 7 |
| 141 | $-CH_3$ | $-CH_3$ | -H | $-CH(CH_3)-CO_2CH_3$ | $-C(=O)-H$ | | 2 |
| 142 | $-CH_3$ | $-CH_3$ | -H | " | $-C(=O)-C_2H_5$ | | 2 |

(Fortsetzung von Tab. 3)

| Beispiel Nr. | $R^1$ | $R^2$ | $R^3$ | R | X | physik.-chem. Kenndaten | Herstellung analog Beisp.-Nr. |
|---|---|---|---|---|---|---|---|
| 143 | $-CH_3$ | $-CH_3$ | -H | 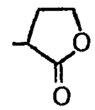 | -H | | 9 |
| 144 | $-CH_3$ | $-CH_3$ | -H | 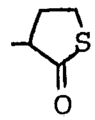 | -H | | 9 |
| 145 | $-CH_3$ | $-CH_3$ | -H | $-CH_2-CO_2CH_3$ | $-\overset{O}{\overset{\|}{C}}-H$ | | 2 |
| 146 | $-CH_3$ | $-CH_3$ | -H | " | $-\overset{O}{\overset{\|}{C}}-CH_3$ | | 2 |
| 147 | $-CH_3$ | $-CH_3$ | -H | 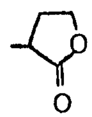 | $-\overset{O}{\overset{\|}{C}}-H$ | | 2 |
| 148 | $-CH_3$ | $-CH_3$ | -H | 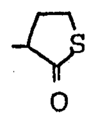 | $-\overset{O}{\overset{\|}{C}}-H$ | | 2 |
| 149 | $-CH_3$ | $-CH_3$ | -H | $-\overset{CH_3}{\overset{\|}{C}H}-COSCH_3$ | $-\overset{O}{\overset{\|}{C}}-H$ | | 2 |
| 150 | $-CH_3$ | $-CH_3$ | -H | $-CH_2-CO_2CH_3$ | -H | | 9 |
| 151 | $-CH_3$ | $-CH_3$ | -H | $-\overset{CH_3}{\overset{\|}{C}H}-CO_2CH_3$ | $-C(=N-CH_2CH_2CH_3)SC_2H_5$ | $n_D^{26,5} = 1,5331$ | 7 |
| 152 | $-CH_3$ | $-CH_3$ | -H | " | $-\underset{O}{\underset{\|}{C}}-CH_2SCH_3$ | | 2 |

Tabelle 4

Optisch aktive Verbindungen.

Formel I mit $R = -\overset{CH_3}{CH}-COR^5$:

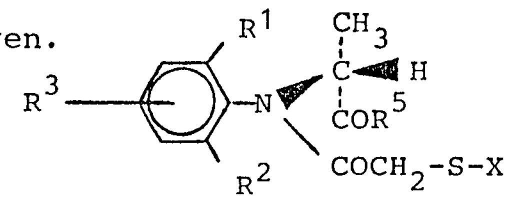

| Beisp. Nr. | $R^1$ | $R^2$ | $R^3$ | $R^5$ | X | physik.-chem. Kenndaten | spez. Drehung $[\alpha]_D^{t°C}$ | Herstellung analog Beisp.-Nr. |
|---|---|---|---|---|---|---|---|---|
| 1 AD | $-CH_3$ | $-CH_3$ | -H | $-OCH_3$ | $-C(=S)-OC_2H_5$ | $n_D^{21} = 1,5630$ | $[\alpha_D]^{20°C} = -24,56°$ | 1 |
| 10 AD | $-CH_3$ | $-CH_3$ | -H | $-OCH_3$ | $-C(=S)-OCH(CH_3)_2$ | $n_D^{20} = 1,5526$ | $[\alpha_D]^{22°C} = -27,46°$ | 1 |
| 9 AD | $-CH_3$ | $-CH_3$ | -H | $-OCH_3$ | -H | | | 9 |
| 5 AD | $-CH_3$ | $-CH_3$ | -H | $-OCH_3$ | $-C(=N-CN)-SCH_3$ | | | 5 |
| 6 AD | $-CH_3$ | $-CH_3$ | -H | $-OCH_3$ | $-C(=S)-NH-CH_3$ | | | 6 |
| 124 AD | $-CH_3$ | $-CH_3$ | -H | $-OCH_3$ | $-C(=N-CH_3)-SCH_3$ | | | 7 |
| 53 AD | $-CH_3$ | $-CH_3$ | -H | $-OC_2H_5$ | $-C(=O)-OCH_3$ | | $[\alpha_D]^{24°C} = -32,53°$ | 4 AD |
| 20 AD | $-CH_3$ | $-CH_3$ | -H | $-OCH_3$ | $-C(=O)-OCH_3$ | Fp.: 76-77°C | $[\alpha_D]^{24°C} = -22,54°$ | 4 AD |

(Fortsetzung von Tab. 4)

| Beisp. Nr. | $R^1$ | $R^2$ | $R^3$ | $R^5$ | X | physik.-chem. Kenndaten | spez. Drehung $[\alpha]_D^{t°C}$ | Herstellung analog Beisp.-Nr. |
|---|---|---|---|---|---|---|---|---|
| 91 AD | $-CH_3$ | $-CH_3$ | -H | $-OCH_3$ | $-C(=S)-SC_2H_5$ | | | 1 |
| 98 AD | $-CH_3$ | $-CH_3$ | -H | $-OCH_3$ | $-C(=O)-SC_2H_5$ | | | 4 AD |
| 2 AD | $-CH_3$ | $-CH_3$ | -H | $-OCH_3$ | $-C(=S)-OCH_3$ | | | 2 |
| 141 AD | $-CH_3$ | $-CH_3$ | -H | $-OCH_3$ | $-C(=O)-H$ | | | 4 AD |
| 104 AD | $-CH_3$ | $-CH_3$ | -H | $-OCH_3$ | $-C(=O)-N(CH_3)_2$ | | | 2 |
| 11 AD | $-CH_3$ | $-CH_3$ | -H | $-OCH_3$ | $-C(=S)-OCH_2CH_2CH_3$ | | | 1 |

B. Formulierungsbeispiele

Beispiel A

Ein Stäubemittel wird erhalten, indem man
10 Gewichtsteile Wirkstoff und
90 Gewichtsteile Talkum als Inertstoff
mischt und in einer Schlagmühle zerkleinert.

Beispiel B

Ein in Wasser leicht dispergierbares, benetzbares Pulver wird erhalten, indem man
25 Gewichtsteile Wirkstoff
64 Gewichtsteile kaolinhaltigen Quarz als Inertstoff
10 Gewichtsteile ligninsulfonsaures Natrium und
1 Gewichtsteil oleoylmethyltaurinsaures Natrium als Netz- und Dispergiermittel
mischt und in einer Stiftmühle mahlt.

Beispiel C

Ein in Wasser leicht dispergierbares Dispersionskonzentrat wird erhalten, indem man
20 Gewichtsteile Wirkstoff mit
6 Gewichtsteilen Nonylphenolpolyglykoläther (10 AeO)
3 Gewichtsteilen Isotridecanolpolyglykoläther (8 AeO) und
71 Gewichtsteilen paraffinischem Mineralöl (Siedebereich z.B. ca. 255 bis über 377°C/Normaldruck)
mischt und in einer Reibkugelmühle auf eine Feinheit von unter 5 Mikron vermahlt.

Beispiel D

Ein emulgierbares Konzentrat wird erhalten aus
15 Gewichtsteilen Wirkstoff
75 Gewichtsteilen Cyclohexanon als Lösungsmittel und
10 Gewichtsteilen oxäthyliertem Nonylphenol (10 AeO)
als Emulgator.

Beispiel E

Ein 5 Gew.-% Wirkstoff enthaltendes Granulat wird erhalter aus

5 Gewichtsteilen Wirkstoff
0,25 Gewichtsteilen Epichlorhydrin
0,25 Gewichtsteilen Cetylpolyglykoläther
3,5 Gewichtsteilen Polyäthylenglykol und
91 Gewichtsteilen Kaolin,

indem man den Wirkstoff mit dem Epichlorhydrin vermischt, das Gemisch in 6 Gewichtsteilen Aceton löst und dann das Polyäthylenglykol sowie den Cetylpolyglykoläther zusetzt. Die resultierende Lösung wird auf das Kaolin der gewünschten Korngröße aufgesprüht und vom Kaolin adsorbiert. Anschließend wird das Aceton unter Vakuum verdampft.

## C. Biologische Beispiele

### Beispiel I

Wirkung der beanspruchten Verbindungen gegen Phytophthora infestans auf Solanum lycopersicum

a) Präventive Wirkung

Tomatenpflanzen (Solanum lycopersicum) der Sorte Rheinlandsruhm werden im 3-Blattstadium mit den nach den Beispielen 1 bis 152 hergestellten Verbindungen der Formel I in einer Wirkstoffkonzentration von jeweils 500 mg/Liter Spritzbrühe tropfnaß gespritzt. Nach dem Antrocknen des Spritzbelags werden die Pflanzen mit einer Zoosporangiensuspension von Phytophthora infestans stark inokuliert und einen Tag lang tropfnaß in eine Klimakammer mit einer Temperatur von 16 °C und einer relativen Luftfeuchte von fast 100 % gestellt. Anschließend kommen sie in ein Kühlgewächshaus mit einer Temperatur von 15 °C und einer relativen Luftfeuchtigkeit von 90 - 95 %. Nach einer Inkubationszeit von 7 Tagen werden die Pflanzen auf Befall mit Phytophthora untersucht. Anzahl und Größe der typischen Phytophthora-Blattflecken dienen als Bewertungsmaßstab für die Wirksamkeit der beanspruchten Verbindungen. Der Befallsgrad wird ausgedrückt in % befallener Blattfläche, vergleichsweise zu unbehandelten, infizierten Kontrollpflanzen.

Die Verbindungen der Beispiele 1 bis 152 bewirken eine Reduktion des Pilzbefalls auf durchschnittlich unter 20 %. Eine Reihe von Verbindungen der Formel I ergibt bei noch wesentlich niedrigeren Aufwandmengen eine nahezu vollständige Reduktion des Pilzbefalls (0-5 %), wie nachfolgend in Tabelle Ia das Ergebnis mit den dort angegebenen Verbindungen der Formel I zeigt.

Tabelle Ia

| Verbindungen gemäß Beispiel Nr. | % Phytophthora-Befall bei mg Wirkstoff/Ltr. Spritzbrühe | | | | |
|---|---|---|---|---|---|
| | 500 | 250 | 125 | 60 | 30 |
| 1 | 0 | 0 | 0 | 0 | 0 |
| 10 | 0 | 0 | 0 | 0 | |
| 13 | 0 | 0 | 0 | 0 | |
| 86 | 0 | 0 | 0 | | |
| 88 | 0 | 0 | 0 - 3 | | |
| 12 | 0 | 0 | 0 | | |
| 36 | 0 | 0 | 0 | | |
| 48 | 0 | 0 | 0 | | |
| 11 | 0 | 0 | 0 | | |
| 15 | 0 | 0 | 0 | | |
| 16 | 0 | 0 | 0 | | |
| 21 | 0 | 0 | 0 - 3 | | |
| 45 | 0 | 0 | 0 | | |
| 39 | 0 | 0 | 0 | | |
| 91 | 0 | 0 | 0 | | |
| 96 | 0 | 0 | 0 | | |
| 1 AD | 0 | 0 | 0 | | |
| 19 | 0 | 0 | 0 | | |
| 20 | 0 | 0 | 0 | | |
| 101 | 0 | 0 | 0 | | |
| 102 | 0 | 0 | 0 | | |
| 124 | 0 | 0 | 0 | | |
| 104 | 0 | 0 | 0 | | |

| Verbindungen gemäß Beispiel Nr. | % Phytophthora-Befall bei mg Wirkstoff/Ltr. Spritzbrühe | | | | |
|---|---|---|---|---|---|
| | 500 | 250 | 125 | 60 | 30 |
| 8 | 0 | 0 | 0 | | |
| 98 | 0 | 0 | 0 | | |
| unbeh. infiz. Pflanzen | 100 | | | | |

b) Curative Wirkung

Tomatenpflanzen (Solanum lycopersicum) der Sorte Rheinlandsruhm werden im 3-Blattstadium mit einer Zoosporangiensuspension von Phytophthora infestans stark inokuliert und 24 Stunden in einer Klimakammer bei einer Temperatur von 15°C und einer relativen Luftfeuchtigkeit von nahezu 100 % inkubiert. Nach dem Abtrocknen der Pflanzen werden diese mit den gemäß den Beispielen 1 bis 152 hergestellten und als Spritzpulver formulierten Verbindungen der Formel I bei Wirkstoffkonzentrationen in den Spritzbrühen von jeweils 500 ppm besprüht. Anschließend werden die Pflanzen wieder in ein Gewächshaus mit einer Temperatur von ca. 16°C und einer relativen Luftfeuchtigkeit von ca. 90 - 95 % gestellt. Fünf Tage danach werden die Pflanzen auf Befall mit Phytophthora infestans untersucht. Anzahl und Größe der typischen Phytophthora-Blattflecken dienen als Maßstab für die Bewertung der Wirksamkeit der geprüften Verbindungen.

Der Befallsgrad wird ausgedrückt in % befallener Blattfläche, vergleichsweise zu unbehandelten, infizierten Kontrollpflanzen.

Die Verbindungen der Beispiele 1 bis 152 bewirken eine Reduktion des Phytophtorabefalls auf durchschnittlich unter 20 %. Eine Reihe von Verbindungen der Formel I ergibt bei noch wesentlich niedrigeren Aufwandmengen eine praktisch vollständige Reduktion des Pilzbefalls, wie nachstehend in der Tabelle Ib das Ergebnis mit den dort angegebenen Verbindungen der Formel I zeigt.

Tabelle Ib

| Verbindungen gemäß Beispiel Nr. | % Phytophthora-Befall bei mg Wirkstoff/Ltr. Spritzbrühe 500 | 250 | 125 | 60 |
|---|---|---|---|---|
| 1 (als Spritzpulver formuliert) | 0 | 0 | 0 | 0 |
| 1 (als Emulsionskonzentrat formuliert) | 0 | 0 | 0 | 0 |
| 13 (als Spritzpulver formuliert) | 0 | 0 | 0 | 0 |
| 5 | 0 | 0 | 0 | |
| 1 AD | 0 | 0 | 0 | 0 |
| 91 | 0 | 0 | 0 | |
| 20 | 0 | 0 | 0 | |
| 7 | 0 | 0 | 0 | |
| unbeh., infiz. Pflanzen | 100 | | | |

c) Präventiv-systemische Wirkung

Die nach den Beispielen 1 bis 152 hergestellten und als Spritzpulver formulierten Verbindungen der Formel I werden in Wirkstoffkonzentrationen von jeweils 200 ppm (bezogen auf das Bodenvolumen) auf die Bodenoberfläche von ca. 3-4 Wochen alten, eingetopften Tomatenpflanzen der Sorte Rheinlandsruhm gegeben. Drei Tage später werden die Pflanzen mit einer Zoosporangiensuspension von Phytophthora infestans inokuliert und, wie unter

Beispiel Ia beschrieben, inkubiert. Nach einer Inkubationszeit von 6-7 Tagen werden die Pflanzen auf Befall mit Phytophthora infestans untersucht. Anzahl und Größe der auf den Blättern gebildeten typischen Phytophthora-Blattflecken bilden den Maßstab für die Beurteilung der Wirksamkeit der geprüften Verbindungen.

Der Befallsgrad wird ausgedrückt in % befallener Blattfläche, vergleichsweise zu unbehandelten, infizierten Kontrollpflanzen.

Die Verbindungen der Beispiele 1 bis 152 bewirken eine Reduktion des des Pilzbefalls auf durchschnittlich unter 20 %. Eine Reihe von Verbindungen der Formel I ergibt bei noch wesentlich niedrigeren Aufwandmengen eine praktisch vollständige Unterdrückung des Pilzbefalls, wie nachstehend in der Tabelle Ic das Ergebnis mit den dort angegebenen Verbindungen der Formel I zeigt.

Tabelle Ic

| Verbindungen gemäß Beispiel Nr. | % Phytophthora-Befall bei ppm Wirkstoff im Boden | | | |
|---|---|---|---|---|
| | 200 | 100 | 50 | 25 |
| 1 | 0 | 0 | 0 | 0 |
| 13 | 0 | 0 | 0 | 0 |
| 91 | 0 | 0 | 0 | |
| 20 | 0 | 0 | 0 | |
| 4 AD | 0 | 0 | 0 | |
| unbeh., infiz. Pflanzen | 100 | | | |

Beispiel II

Wirkung gegen Plasmopara viticola auf Reben

a) Präventive Wirkung

Weinpflanzen, die aus Stecklingen der für Plasmopara anfälligen Sorte Müller Thurgau gezogen wurden, werden im 4-Blattstadium mit wässrigen Suspensionen der gemäß den Beispielen 1 bis 152 hergestellten Verbindungen der Formel I in einer Wirkstoffkonzentration von jeweils 500 mg/Ltr. Spritzbrühe tropfnaß gespritzt. Nach dem Antrocknen des Spritzbelags werden die Pflanzen mit einer Zoosporangiensuspension von Plasmopara viticola inokuliert und tropfnaß in eine Klimakammer mit einer Temperatur von 20$^{o}$C und einer relativen Luftfeuchte von ca. 100 % gestellt. Nach 24 Stunden werden die infizierten Pflanzen der Klimakammer entnommen und in ein Gewächshaus mit einer Temperatur von 23$^{o}$C und einer Luftfeuchtigkeit von ca. 98 % gebracht. Nach einer Inkubationszeit von 7 Tagen werden die Pflanzen angefeuchtet, über Nacht in die Klimakammer gestellt und die Krankheit zum Ausbruch gebracht. Anschließend erfolgt die Befallsauswertung. Anzahl und Größe der Infektionsstellen auf den Blättern der inokulierten und behandelten Pflanzen dienen als Maßstab für die Wirksamkeit der geprüften Verbindungen. Der Befallsgrad wird in % befallener Blattfläche, vergleichsweise zu unbehandelten, infizierten Kontrollpflanzen ausgedrückt.

Mit den Verbindungen der Beispiele 1 bis 152 wird eine weitgehende Unterdrückung des Pilzbefalls erreicht (durchschnittlicher Befallsgrad $<$20 %). Eine Reihe von Verbindungen der Formel I ergibt bei noch wesentlich niedrigeren Anwendungskonzentrationen eine praktisch vollständige Unterdrückung des Pilzbefalls, wie nachstehend in der Tabelle II das Ergebnis mit den dort angegebenen Verbindungen der Formel I zeigt.

Tabelle II

| Verbindungen gemäß Beispiel Nr. | % Plasmopara-Befall bei mg Wirkstoff/Ltr. Spritzbrühe 500 | 250 | 125 | 60 | 30 |
|---|---|---|---|---|---|
| 1 | 0 | 0 | 0 | 0 | 0 |
| 10 | 0 | 0 | 0 | 0 | 0 |
| 13 | 0 | 0 | 0 | 0 | 0 |
| 86 | 0 | 0 | 0 | | |
| 89 | 0 | 0 | 0 | | |
| 71 | 0 | 0 | 0 | | |
| 12 | 0 | 0 | 0 | | |
| 22 | 0 | 0 | 0 | | |
| 36 | 0 | 0 | 0 | | |
| 48 | 0 | 0 | 0 | | |
| 11 | 0 | 0 | 0 | | |
| 15 | 0 | 0 | 0 | | |
| 37 | 0 | 0 | 0 | | |
| 16 | 0 | 0 | 0 | | |
| 21 | 0 | 0 | 0 | | |
| 24 | 0 | 0 | 0 | | |
| 45 | 0 | 0 | 0 | | |
| 46 | 0 | 0 | 0 | | |
| 4 AD | 0 | 0 | 0 | | |
| 20 AD | 0 | 0 | 0 | | |
| 19 | 0 | 0 | 0 | | |
| 47 | 0 | 0 | 0 | | |
| 1 AD | 0 | 0 | 0 | | |
| 38 | 0 | 0 | 0 | | |
| 39 | 0 | 0 | 0 | | |
| 91 | 0 | 0 | 0 | | |
| 96 | 0 | 0 | 0 | | |
| 42 | 0 | 0 | 0 | | |
| 51 | 0 | 0 | 0 | | |
| 101 | 0 | 0 | 0 | | |
| 102 | 0 | 0 | 0 | | |
| 104 | 0 | 0 | 0 | | |
| 124 | 0 | 0 | 0 | | |

Forts. Tabelle II

| Verbindungen gemäß Beispiel Nr. | % Plasmopara-Befall bei mg Wirkstoff/Ltr. Spritzbrühe 500 | 250 | 125 | 60 | 30 |
|---|---|---|---|---|---|
| 6 | 0 | 0 | 0 | | |
| 142 | 0 | 0 | 0 | | |
| 9 | 0 | 0 | 0 | | |
| 98 | 0 | 0 | 0 | | |
| unbeh., infiz. Pflanzen | 100 | | | | |

Beispiel III

Wirkung gegen Pythium- ultimum an Erbsen

a) Wirkung nach Bodenapplikation

Der Pilz wird in einer Sandkultur angezogen und mit Erde vermischt. Die so infizierte Erde wird als Kulturboden in Anzuchttöpfe abgefüllt und mit Erbsensaatgut besät. Nach der Aussaat werden die gemäß den Beispielen 1 bis 152 hergestellten Verbindungen der Formel I als wässrige Suspensionen in einer Wirkstoffkonzentration von jeweils 200 ppm (bezogen auf das Bodenvolumen) auf die Bodenoberfläche gegossen. Die Töpfe werden anschließend 2-3 Wochen in einem Gewächshaus bei 20-22°C aufgestellt und der Boden gleichmäßig leicht feuchtgehalten. Bei der Auswertung auf Pythium-Befall wird der Auflauf der Erbsenpflänzchen sowie der Anteil gesunder und kranker Pflanzen, vergleichsweise zu den entsprechenden Kontrollen ermittelt und daraus der Wirkungsgrad der geprüften Verbindungen errechnet.

Mit den Verbindungen der Beispiele 1 bis 152 wird eine weitgehende Reduktion des Pythium- ultimum-Befalls, wobei der Wirkungsgrad durchschnittlich > 80 % beträgt, erzielt.

Eine Reihe von Verbindungen der Formel I bewirkt bei noch wesentlich niedrigeren Wirkstoffkonzentrationen eine praktisch vollständige Unterdrückung des Pilzbefalls, wie nachstehend in der Tabelle III die Ergebnisse mit den dort angegebenen Verbindungen der Formel I zeigen.

Tabelle III

Fungizide Wirkung gegen Pythium- ultimum nach Behandlung von beimpftem Kulturboden mit den genannten Verbindungen der Formel I als Wirkstoff in den angegebenen Konzentrationen und anschließender Aussaat von gesundem Erbsensaatgut.

1. Tag: Beimpfung und Behandlung des Bodens mit Wirkstoff, Aussaat;

14. Tag: Auswertung

| Verbindungen gemäß Beispiel Nr. | Fungizider Wirkungsgrad in % bei ppm Wirkstoff im Boden 200 | 100 | 50 | 25 |
|---|---|---|---|---|
| 1 | 100 | 100 | 100 | 100 |
| 86 | 100 | 100 | | |
| 12 | 100 | 100 | 100 | |
| 1 AD | 100 | 100 | 100 | |
| 4 AD | 100 | 100 | 100 | |
| 20 | 100 | 100 | 100 | |
| 36 | 100 | 100 | | |
| 48 | 100 | 100 | 100 | |
| 11 | 100 | 100 | 100 | |
| 15 | 100 | 100 | 100 | |
| 38 | 100 | 100 | | |
| 39 | 100 | 100 | | |
| 91 | 100 | 100 | 100 | |
| 96 | 100 | 100 | | |
| 10 AD | 100 | 100 | 100 | |

(Fortsetzg.v. Tab. III)



| Verbindungen gemäß Beispiel Nr. | Fungizider Wirkungsgrad in % bei ppm Wirkstoff im Boden 200 | 100 | 50 | 25 |
|---|---|---|---|---|
| 10 | 100 | 100 | 100 | |
| 98 | 100 | 100 | 100 | |
| 102 | 100 | 100 | | |
| 104 | 100 | 100 | 100 | |
| 115 | 100 | 100 | | |
| 124 | 100 | 100 | 100 | |
| Vergleichs-mittel A*) | 65 | 40 | 0 | |
| unbehandelter, beimpfter Boden | 0 (= 100 % Auflaufschäden) | | | |
| unbehandelter, nicht beimpfter Boden | 100 % Auflauf | | | |

*) Vergleichsmittel A: Methyl-2-/N-(methylthioacetyl)-N-(2,6-dimethylphenyl)-amino/-propionat (vgl. DE-OS 25 15 091).

Beispiel IV

Präventive Wirkung der Verbindungen der Formel I gegen Phytophthora capsici (Bodenapplikation).

Phytophthora capsici wird in Sandkulturen angezogen und mit Versuchserde vermischt. Der so infizierte Boden wird in Anzuchttöpfe abgefüllt und mit Paprika (Capsicum annuum) besät. Nach der Aussaat werden die beanspruchten Verbindungen als wäßrige Suspensionen auf die Bodenoberfläche gegossen oder in die Abdeckerde eingearbeitet. Dabei werden Wirkstoffkonzentrationen von 200 bis 50 ppm (bezogen auf das Bodenvolumen) angewandt. Die Töpfe werden anschließend ca. 3 Wochen in ein Gewächshaus bei 22 - 24 °C gestellt und der Kulturboden gleichmäßig feucht gehalten. Bei der Auswertung auf Phytophthora capsici-Befall werden der Auflauf der Paprika-Pflanzen sowie der Anteil gesunder und kranker Pflanzen im Vergleich zu den entsprechenden Kontrollen ermittelt und entsprechend der Wirkungsgrad errechnet.

Die Verbindungen der Formel I ergeben im allgemeinen bei einer Aufwandmenge von 200 ppm eine vollständige Kontrolle des Phytophthora capsici-Befalls. Eine Reihe von Verbindungen der Formel I führt bei noch wesentlich geringerer Aufwandmenge zu einer ebenfalls vollständigen Reduktion des Pilzbefalls, wie nachfolgend die in der Tabelle IV zusammengefaßten Versuchsergebnisse zeigen.

Tabelle IV

Wirkung gegen Phytophthora capsici nach Behandlung von beimpfter Erde mit fungiziden Verbindungen der Formel I und Aussaat von gesundem Paprika-Saatgut.

1. Tag: Beimpfung des Bodens und Behandlung mit fungizider Verbindung, Aussaat

Ca. 21. Tag: Auswertung

| Verbindung gem. Beispiel Nr. | Wirkungsgrad in % bei ppm Wirkstoff im Boden | | |
|---|---|---|---|
| | 200 ppm | 100 ppm | 50 ppm |
| 12 | 100 | 100 | 100 |
| 36 | 100 | 100 | 100 |
| 11 | 100 | 100 | 100 |
| 15 | 100 | 100 | 95 |
| 91 | 100 | 100 | 100 |
| 96 | 100 | 100 | 100 |
| 1 | 100 | 100 | 100 |
| 10 | 100 | 100 | 100 |
| 1 AD | 100 | 100 | 100 |

(Fortsetzg.v. Tab. IV) 

| Verbindung gem. Beispiel Nr. | Wirkungsgrad in % bei ppm Wirkstoff im Boden 200 ppm | 100 ppm | 50 ppm |
|---|---|---|---|
| 20 | 100 | 100 | 100 |
| 20 AD | 100 | 100 | 100 |
| 98 | 100 | 100 | 100 |
| 104 | 100 | 100 | 100 |
| 124 | 100 | 100 | 100 |
| Kontrolle: | | | |
| unbehandelte, beimpfte Erde | 0 (= 100 % Auflaufschäden) | | |
| unbehandelte, nicht beimpfte Erde | 100 % Auflauf | | |

Patentansprüche:

1. Verbindungen der allgemeinen Formel I,

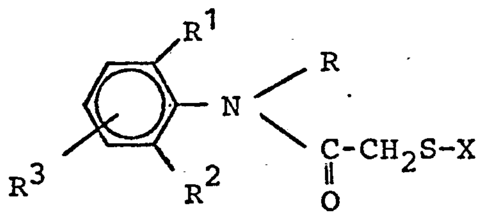

(I)

worin

R = $-\overset{R^4}{\overset{|}{C}}HCOR^5$, 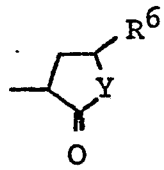

X = 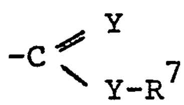 , 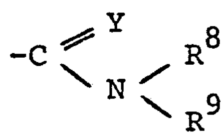 , 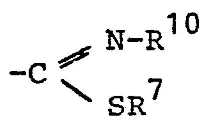

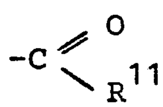 , Wasserstoff

$R^1$ = $(C_1-C_4)$-Alkyl, Halogen, $(C_1-C_3)$-Alkylthio, $(C_1-C_4)$-Alkoxy, $(C_3-C_4)$-Alkenyl

$R^2$ = Wasserstoff, $(C_1-C_4)$-Alkyl, Halogen

$R^3$, $R^4$, $R^6$ = Wasserstoff, Methyl

$R^5$ = Hydroxy, $(C_1-C_4)$-Alkoxy, $(C_1-C_3)$-Alkylthio, $(C_1-C_2)$-Alkoxy-$(C_1-C_2)$-alkoxy, Amino, $(C_1-C_2)$-Alkylamino, Di-$(C_1-C_2)$-alkylamino

Y = Sauerstoff oder Schwefel

$R^7$ = $(C_1-C_6)$-Alkyl, welches durch $(C_1-C_2)$-Alkoxy oder Halogen substituiert sein kann, $(C_3-C_6)$-Cycloalkyl, $(C_3-C_4)$-Alkenyl, $(C_3-C_4)$-Alkinyl.

$R^8$ = Wasserstoff, $(C_1-C_4)$-Alkyl

$R^9$ = $(C_1-C_4)$-Alkyl, welches gegebenenfalls durch Halogen, $(C_1-C_2)$-Alkoxy substituiert ist, $(C_3-C_4)$-Alkenyl, Furfuryl, Tetrahydrofurfuryl, mit der Maßgabe, daß für Y=S nicht beide Reste $R^8$ und $R^9$ $(C_1-C_4)$-Alkyl bedeuten

$R^{10}$ = -CN, $(C_1-C_4)$-Alkyl, welches gegebenenfalls durch Halogen oder $(C_1-C_2)$-Alkoxy substituiert ist, Allyl, Furfuryl

$R^{11}$ = Wasserstoff, gegebenenfalls durch ein- bis drei Halogenatome, $(C_1-C_2)$-Alkoxy oder $(C_1-C_2)$-Alkylthio substituiertes $(C_1-C_4)$-Alkyl, $(C_2-C_4)$-Alkenyl, mit der Maßgabe, daß $R^{11}$ im Falle von R = 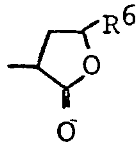 nicht für $(C_1-C_4)$-Alkyl oder halogen-substituiertes $(C_1-C_4)$-Alkyl steht, bedeutet.

2. Verbindungen gemäß Anspruch 1, dadurch gekennzeichnet, daß in Formel I

R = 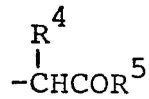 , 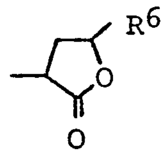

X = 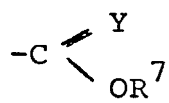

$R^1$= $(C_1-C_4)$-Alkyl, Halogen

$R^2$= H, $(C_1-C_4)$-Alkyl, Halogen

$R^3$, $R^4$, $R^6$= Wasserstoff, Methyl

$R^7$= $(C_1-C_6)$-Alkyl, welches durch $(C_1-C_4)$-Alkoxy oder Halogen substituiert sein kann, $(C_3-C_6)$-Cycloalkyl, $(C_3-C_4)$-Alkenyl, $(C_3-C_4)$-Alkinyl

$R^5$= Hydroxy, $(C_1-C_4)$-Alkoxy, $(C_1-C_3)$-Alkylthio, Amino, $(C_1-C_2)$-Alkylamino, Di-$(C_1-C_2)$-alkylamino, $(C_1-C_2)$-Alkoxyäthoxy ,

Y = O,S

bedeutet.

3. Verbindungen gemäß Anspruch 1, dadurch gekennzeichnet, daß in Formel I

R = 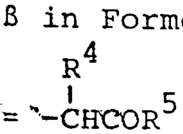 , 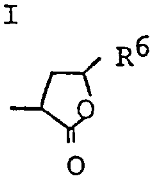

X = 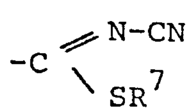

bedeutet und $R^1$, $R^2$, $R^3$, $R^4$, $R^5$, $R^6$ und $R^7$ die Bedeutungen wie in Anspruch 1 haben.

4. Verfahren zur Herstellung von Verbindungen der Formel I gemäß Ansprüchen 1 bis 3, dadurch gekennzeichnet, daß man

a) Verbindungen der allgemeinen Formel II,

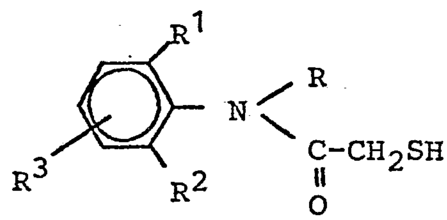 (II)

in der R, $R^1$, $R^2$ und $R^3$ die Bedeutungen wie in Formel I haben, in Abhängigkeit vom Rest X in Formel I mit

$a_1$) Säurehalogeniden der allgemeinen Formel III,

Hal–X (III)

in der X die Bedeutung wie in Formel I hat und Hal für Halogen, vorzugsweise Chlor oder Brom steht, umsetzt, gegebenenfalls in inerten Lösungs- oder Verdünnungsmitteln und gegebenenfalls unter Zusatz von säurebindenden Mitteln, oder daß man

$a_2$) zur Herstellung von Verbindungen der Formel I

mit X = 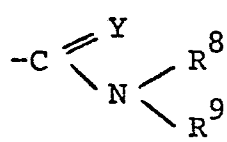 und $R^8$ = H, Verbindungen der Formel II mit Isocyanaten der Formel IIIb,

$$YCN - R^9 \qquad \text{(IIIb)}$$

worin Y und $R^9$ die Bedeutungen wie in Formel I haben, umsetzt, gegebenenfalls in inerten Lösungs- oder Verdünnungsmitteln und gegebenenfalls unter Zugabe von Katalysatoren, oder daß man

b) Verbindungen der allgemeinen Formel IV,

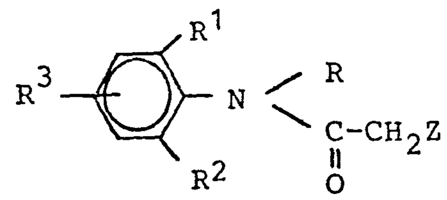

(IV)

in der R, $R^1$, $R^2$, $R^3$ die Bedeutungen wie in Formel I haben und Z einen nukleophil substituierbaren Abgangsrest, vorzugsweise aus der Gruppe Halogen, Tosylat oder Mesylat, insbesondere Chlor oder Brom, bedeutet, mit Verbindungen der allgemeinen Formel V,

MeS - X (V)

in der X die Bedeutung wie in Formel I hat und Me ein anorganisches oder organisches Kation, vorzugsweise Natrium, Kalium, 1/2-Calcium, 1/2-Magnesium oder Ammonium bedeutet, umsetzt, gegebenenfalls in inerten Lösungs- oder Verdünnungsmitteln, oder daß man

c) Anilinderivate der allgemeinen Formel VI,

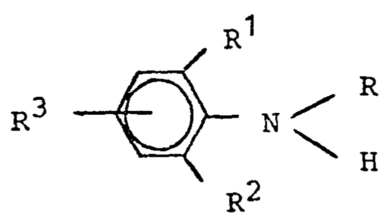

(VI)

in der R, $R^1$, $R^2$ und $R^3$ die Bedeutungen wie in Formel I haben, mit Säurehalogeniden oder Säureanhydriden der allgemeinen Formel VII,

$$W-\overset{\overset{O}{\|}}{C}-CH_2-SX \qquad \text{(VII)}$$

in der X die Bedeutung wie in Formel I hat und W ein Halogenatom, vorzugsweise Chlor oder Brom, oder den Rest $XS-CH_2-\overset{O}{C}-O-$ , worin X die Bedeutung wie in Formel VII hat, bedeutet, umsetzt, gegebenenfalls in inerten Lösungs- oder Verdünnungsmitteln und gegebenenfalls unter Zusatz von säurebindenden Mitteln, oder daß man

d) zur Herstellung von Verbindungen der Formel I mit

X = 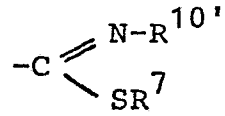 ,

Verbindungen der allgemeinen Formel VIII,

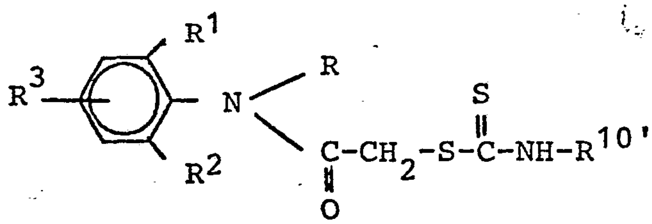 (VIII)

in der R, $R^1$, $R^2$ und $R^3$ die Bedeutungen wie in Formel I haben und $R^{10'}$ für $(C_1-C_4)$-Alkyl, welches gegebenenfalls durch $(C_1-C_2)$-Alkoxy substituiert ist, Allyl oder Furfuryl steht, mit Halogeniden oder Sulfaten der Formel IX,

$$Q - R^7 \qquad (IX)$$

in der $R^7$ die Bedeutung wie in Formel I hat und Q Halogen oder $R^7O-\overset{O}{\underset{O}{S}}-O-$ , vorzugsweise Chlor, Brom oder Jod, bedeutet, umsetzt, gegebenenfalls in inerten Lösungs- oder Verdünnungsmitteln und gegebenenfalls unter Zusatz von säurebindenden Mitteln.

5. Fungizide Mittel, gekennzeichnet durch einen Gehalt an einer Verbindung der Formel I gemäß Ansprüchen 1 bis 3 als Wirkstoff.

6. Fungizide Mittel, gekennzeichnet durch einen Gehalt von 2 bis 95 Gew.-%, vorzugsweise 5 bis 90 Gew.-% an einer Verbindung der Formel I gemäß Ansprüchen 1 bis 3 als Wirkstoff sowie üblichen Formulierungshilfsmitteln.

7. Verwendung von Verbindungen der Formel I gemäß Ansprüchen 1 bis 3, 5 und 6 zur Schädlingsbekämpfung im Pflanzenschutz.

8. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1 bis 3, 5 und 6 in Konakt bringt.

Patentansprüche für Österreich:

1. Verfahren zur Herstellung von Verbindungen der allgemeinen Formel I,

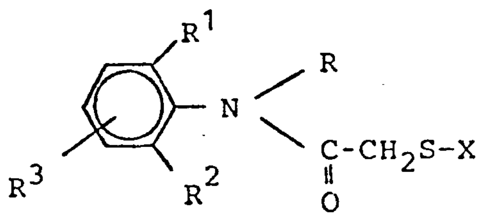

(I)

worin

R = 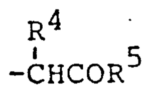, 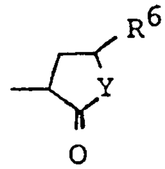

X = 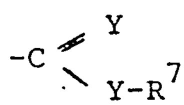, 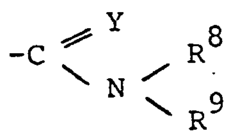, 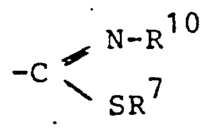

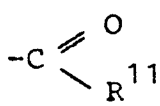, Wasserstoff

$R^1$ = $(C_1-C_4)$-Alkyl, Halogen, $(C_1-C_3)$-Alkylthio, $(C_1-C_4)$-Alkoxy, $(C_3-C_4)$-Alkenyl

$R^2$ = Wasserstoff, $(C_1-C_4)$-Alkyl, Halogen

$R^3$, $R^4$, $R^6$ = Wasserstoff, Methyl

$R^5$ = Hydroxy, $(C_1-C_4)$-Alkoxy, $(C_1-C_3)$-Alkylthio, $(C_1-C_2)$-Alkoxy-$(C_1-C_2)$-alkoxy, Amino, $(C_1-C_2)$-Alkylamino, Di-$(C_1-C_2)$-alkylamino

Y = Sauerstoff oder Schwefel

$R^7$ = $(C_1-C_6)$-Alkyl, welches durch $(C_1-C_2)$-Alkoxy oder Halogen substituiert sein kann, $(C_3-C_6)$-Cycloalkyl, $(C_3-C_4)$-Alkenyl, $(C_3-C_4)$-Alkinyl

$R^8$ = Wasserstoff, $(C_1-C_4)$-Alkyl

$R^9$ = $(C_1-C_4)$-Alkyl, welches gegebenenfalls durch Halogen, $(C_1-C_2)$-Alkoxy substituiert ist, $(C_3-C_4)$-Alkenyl, Furfuryl, Tetrahydrofurfuryl, mit der Maßgabe, daß für Y=S nicht beide Reste $R^8$ und $R^9$ $(C_1-C_4)$-Alkyl bedeuten

$R^{10}$ = -CN, $(C_1-C_4)$-Alkyl, welches gegebenenfalls durch Halogen oder $(C_1-C_2)$-Alkoxy substituiert ist, Allyl, Furfuryl

$R^{11}$ = Wasserstoff, gegebenenfalls durch ein- bis drei Halogenatome, $(C_1-C_2)$-Alkoxy oder $(C_1-C_2)$-Alkylthio substituiertes $(C_1-C_4)$-Alkyl, $(C_2-C_4)$-Alkenyl, mit der Maßgabe, daß $R^{11}$ im Falle von

R = 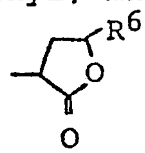 nicht für $(C_1-C_4)$-Alkyl oder halogensubstituiertes $(C_1-C_4)$-Alkyl steht, bedeutet,

dadurch gekennzeichnet, daß man

a) Verbindungen der allgemeinen Formel II,

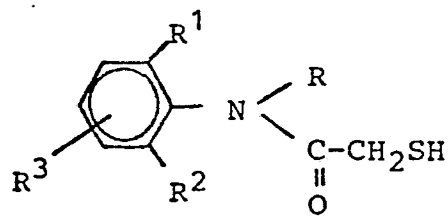

(II)

in der R, $R^1$, $R^2$ und $R^3$ die Bedeutungen wie in Formel I haben, in Abhängigkeit vom Rest X in Formel I mit

$a_1$) Säurehalogeniden der allgemeinen Formel III,

Hal-X (III)

in der X die Bedeutung wie in Formel I hat und Hal für Halogen, vorzugsweise Chlor oder Brom steht, umsetzt, gegebenenfalls in inerten Lösungs- oder Verdünnungsmitteln und gegebenenfalls unter Zusatz von säurebindenden Mitteln, oder daß man

$a_2$) zur Herstellung von Verbindungen der Formel I

mit X = 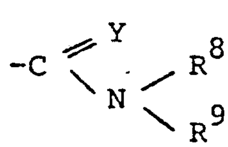 und $R^8$ = H, Verbindungen

der Formel II mit Isocyanaten der Formel IIIb,

YCN - $R^9$ (IIIb)

worin Y und $R^9$ die Bedeutungen wie in Formel I haben, umsetzt, gegebenenfalls in inerten Lösungs- oder Verdünnungsmitteln und gegebenenfalls unter Zugabe von Katalysatoren, oder daß man

b) Verbindungen der allgemeinen Formel IV,

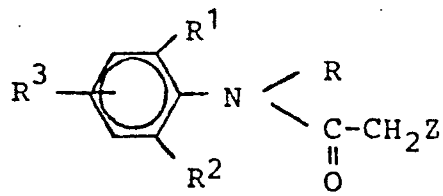

(IV)

in der R, $R^1$, $R^2$, $R^3$ die Bedeutungen wie in Formel I haben und Z einen nukleophil substituierbaren Abgangsrest, vorzugsweise aus der Gruppe Halogen, Tosylat oder Mesylat, insbesondere Chlor oder Brom, bedeutet, mit Verbindungen der allgemeinen Formel V,

MeS - X (V)

in der X die Bedeutung wie in Formel I hat und Me ein anorganisches oder organisches Kation, vorzugsweise Natrium, Kalium, 1/2-Calcium, 1/2-Magnesium oder Ammonium bedeutet, umsetzt, gegebenenfalls in inerten Lösungs- oder Verdünnungsmitteln, oder daß man

c) Anilinderivate der allgemeinen Formel VI,

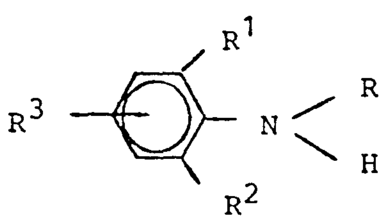

(VI)

in der R, $R^1$, $R^2$ und $R^3$ die Bedeutungen wie in Formel I haben, mit Säurehalogeniden oder Säureanhydriden der allgemeinen Formel VII,

$$W-\overset{O}{\overset{\|}{C}}-CH_2-SX \qquad \text{(VII)}$$

in der X die Bedeutung wie in Formel I hat und W ein Halogenatom, vorzugsweise Chlor oder Brom, oder den Rest $XS-CH_2-\overset{O}{\overset{\|}{C}}-O-$ , worin X die Bedeutung wie in Formel VII hat, bedeutet, umsetzt, gegebenenfalls in inerten Lösungs- oder Verdünnungsmitteln und gegebenenfalls unter Zusatz von säurebindenden Mitteln, oder daß man

d) zur Herstellung von Verbindungen der Formel I mit

X = 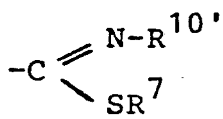 ,

Verbindungen der allgemeinen Formel VIII,

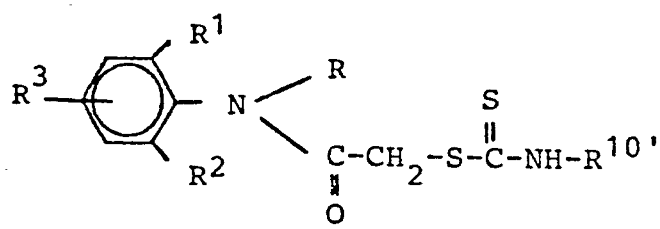
(VIII)

in der R, $R^1$, $R^2$ und $R^3$ die Bedeutungen wie in Formel I haben und $R^{10'}$ für $(C_1-C_4)$-Alkyl, welches gegebenenfalls durch $(C_1-C_2)$-Alkoxy substituiert ist, Allyl oder Furfuryl steht, mit Halogeniden oder Sulfaten der Formel IX,

$$Q - R^7 \qquad \text{(IX)}$$

in der $R^7$ die Bedeutung wie in Formel I hat und Q Halogen oder $R^7O-\underset{O}{\overset{O}{S}}-O-$ , vorzugsweise Chlor, Brom oder Jod, bedeutet, umsetzt, gegebenenfalls in inerten Lösungs- oder Verdünnungsmitteln und gegebenenfalls unter Zusatz von säurebindenden Mitteln.

2. Herstellung von Verbindungen gemäß Anspruch 1, dadurch gekennzeichnet, daß in Formel I

$R = -\overset{\overset{R^4}{|}}{C}HCOR^5$ , 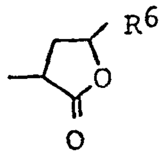

X = 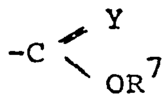

$R^1$ = $(C_1-C_4)$-Alkyl, Halogen
$R^2$ = H, $(C_1-C_4)$-Alkyl, Halogen
$R^3$, $R^4$, $R^6$ = Wasserstoff, Methyl
$R^7$ = $(C_1-C_6)$-Alkyl, welches durch $(C_1-C_4)$-Alkoxy oder Halogen substituiert sein kann, $(C_3-C_6)$-Cycloalkyl, $(C_3-C_4)$-Alkenyl, $(C_3-C_4)$-Alkinyl
$R^5$ = Hydroxy, $(C_1-C_4)$-Alkoxy, $(C_1-C_3)$-Alkylthio, Amino , $(C_1-C_2)$-Alkylamino, Di-$(C_1-C_2)$-alkylamino, $(C_1-C_2)$-Alkoxyäthoxy ,
Y = O,S
bedeutet.

3. Herstellung von Verbindungen gemäß Anspruch 1, dadurch gekennzeichnet, daß in Formel I

$R = -\overset{\overset{R^4}{|}}{C}HCOR^5$ , 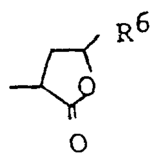

X = 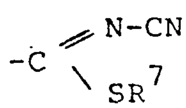

bedeutet und $R^1$, $R^2$, $R^3$, $R^4$, $R^5$, $R^6$ und $R^7$ die Bedeutungen wie in Anspruch 1 haben.

4. Fungizide Mittel, gekennzeichnet durch einen Gehalt an einer Verbindung der Formel I gemäß Ansprüchen 1 bis 3 als Wirkstoff.

5. Fungizide Mittel, gekennzeichnet durch einen Gehalt von 2 bis 95 Gew.-%, vorzugsweise 5 bis 90 Gew.-% an einer Verbindung der Formel I gemäß Ansprüchen 1 bis 3 als Wirkstoff sowie üblichen Formulierungshilfsmitteln.

6. Verwendung von Verbindungen der Formel I gemäß Ansprüchen 1 bis 3, 4 und 5 zur Schädlingsbekämpfung im Pflanzenschutz.

7. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1 bis 3, 4 und 5 in Konakt bringt.